# EUROPÄISCHE PATENTANMELDUNG

(11) **EP 4 089 161 A1**
(43) Veröffentlichungstag der Anmeldung: **16.11.2022**
(21) Anmeldenummer: 21173579.0
(22) Anmeldetag: 12.05.2021
(51) Int. Cl.: C12M 3/06, F16K 99/00, B01L 3/00

(54) **SUBSTRAT ZUR UNTERSUCHUNG VON PROBEN UND SYSTEM UMFASSEND DAS SUBSTRAT**

(71) Anmelder: ibidi GmbH, 82166 Gräfelfing (DE)
(72) Erfinder: Zantl, Roman, Gräfelfing (DE)
(74) Vertreter: Grünecker Patent- und Rechtsanwälte PartG mbB

(57) **Zusammenfassung**

Die Erfindung betrifft ein Substrat zur Untersuchung von Proben, insbesondere Zellen oder Molekülen, wobei das Substrat ein Fluidsystem umfasst, das eine in dem Substrat ausgebildete Probenkammer zur Aufbewahrung und Untersuchung von Proben und mindestens ein mit der Probenkammer fluidisch verbundenes Flüssigkeitsreservoir umfasst, und wobei das Substrat ein passives Sperrelement umfasst, das eine Sperrstellung und eine Durchlassstellung einnehmen kann, wobei in der Sperrstellung ein Flüssigkeitsaustausch zwischen der Probenkammer und dem Flüssigkeitsreservoir gesperrt ist.

## Beschreibung

Die Erfindung betrifft ein Substrat zur Untersuchung von Proben, insbesondere Zellen oder Molekülen, und ein System umfassend das Substrat. Die Erfindung betrifft auch ein Verfahren zur Verwendung des Substrats oder Systems.

Bei der Untersuchung von Proben, insbesondere zur Untersuchung von Zellen oder Beobachtung von chemischen Reaktionen, ist ein wichtiger Anwendungsfall, Flüssigkeit um die Probe herum oder über die Probe strömen zu lassen, beispielsweise Prozessflüssigkeiten oder Reinigungsflüssigkeiten. Dazu werden die Flüssigkeiten in dem Substrat durch ein Fluidsystem einschließlich der Probenkammer transportiert. Häufig werden insbesondere mehrere Flüssigkeiten nacheinander durch das Fluidsystem bewegt.

Bekannte Systeme umfassen in der Regel mehrere außerhalb des Substrats angeordnete Flüssigkeitsbehälter, die an das Substrat angeschlossen werden. Die Behälter können über eine geeignete Kombination aus Anschlüssen und Ventilen mit dem Substrat verbunden sein. Bewegt werden die Flüssigkeiten typischerweise durch Pumpen.

Wenn verschiedene Flüssigkeiten nacheinander durch die Probenkammer transportiert werden sollen, besteht das Risiko einer Durchmischung der Flüssigkeiten in der Probenkammer, außer wenn immer ein vollständiger Austausch der gesamten im Substrat befindlichen Flüssigkeit erfolgt, so dass viel Flüssigkeit und somit auch relativ große Flüssigkeitsbehälter benötigt werden.

Es ist eine Aufgabe der Erfindung, ein Substrat bereitzustellen, das bei geringem Risiko einer Durchmischung von Flüssigkeiten in der Probenkammer einen kompakteren Versuchsaufbau ermöglicht.

Die Aufgabe wird durch den Gegenstand des Anspruchs 1 gelöst. Demnach umfasst das Substrat ein Fluidsystem, das eine in dem Substrat ausgebildete Probenkammer zur Aufbewahrung und Untersuchung von Proben, insbesondere Zellen oder Molekülen, und mindestens ein mit der Probenkammer fluidisch verbundenes Flüssigkeitsreservoir umfasst, und ein passives Sperrelement, das eine Sperrstellung und eine Durchlassstellung einnehmen kann, wobei in der Sperrstellung ein Flüssigkeitsaustausch zwischen der Probenkammer und dem Flüssigkeitsreservoir gesperrt ist.

Mit anderen Worten ist das passive Sperrelement zum Herstellen und Trennen einer fluidischen Verbindung ausgebildet. Das Einnehmen der Sperrstellung wird auch als Schließen bezeichnet und das Einnehmen der Durchlassstellung wird auch als Öffnen bezeichnet. In der Durchlassstellung kann ein Flüssigkeitsaustausch zwischen der Probenkammer und dem Flüssigkeitsreservoir stattfinden, beispielsweise Flüssigkeit von der Probenkammer in das Flüssigkeitsreservoir oder von dem Flüssigkeitsreservoir in die Probenkammer transportiert werden. Die Transportrichtung kann beispielsweise mittels eines an das Fluidsystem angelegten Drucks eingestellt werden.

Das passive Sperrelement ermöglicht einerseits, dass eine zuverlässige Trennung bestimmter im Fluidsystem befindlichen Flüssigkeiten sichergestellt wird, andererseits aber auch, dass bei geeigneter Anwendung Flüssigkeit gezielt zwischen verschiedenen Bereichen des Fluidsystems ausgetauscht werden kann, beispielsweise von der Probenkammer in ein Flüssigkeitsreservoir oder von einem Flüssigkeitsreservoir in die Probenkammer transportiert werden kann. Somit ist es möglich, Prozessflüssigkeiten im Substrat aufzubewahren, bis diese eingesetzt werden sollen, und es müssen auch immer nur geringe Flüssigkeitsmengen ausgetauscht werden, nicht die gesamte im Substrat befindliche Flüssigkeit. Auf die externen Flüssigkeitsbehälter und zugehörigen Pumpen und Ventile kann dann verzichtet werden.

Des passiven Sperrelements ermöglicht dabei hohe Zuverlässigkeit, einen kompakten Aufbau und geringe Materialkosten für das Substrat. Gerade bei Substraten, die als Einwegprodukt vorgesehen sind, ist letzteres vorteilhaft.

Das passive Sperrelement kann ein passives Ventil umfassen, insbesondere ein passives Ventil sein.

Dass es sich um ein passives Sperrelement handelt, kann insbesondere bedeuten, dass das Sperrelement derart ausgebildet ist, dass zum Öffnen und Schließen des Sperrelements, also zum Übergang von einer Durchlassstellung in eine Sperrstellung und zum Übergang von einer Sperrstellung in eine Durchlassstellung, keine aktive Betätigung, insbesondere keine direkte aktive Betätigung, an dem Sperrelement erforderlich ist, beispielsweise keine, insbesondere direkte, Betätigung mittels eines elektrischen oder pneumatischen Antriebs.

Sofern nicht anders spezifiziert, sind im Folgenden alle Angaben, insbesondere die, die sich direkt oder indirekt auf die räumliche Anordnung des Substrats oder Systems beziehen, beispielsweise "oben", "oberhalb", "unten", "unterhalb", "auf einer Höhe mit", "Draufsicht" oder "Seitenansicht", auf die Anordnung des Substrats im bestimmungsgemäßen Gebrauch bezogen.

Dass Teilbereiche des Fluidsystems fluidisch verbunden sind umfasst, dass diese direkt oder indirekt miteinander fluidisch verbunden sind. Wenn Teilbereiche indirekt miteinander fluidisch verbunden sind, können sie beispielsweise über andere Teilbereiche des Fluidsystems miteinander fluidisch verbunden sein. Sie können insbesondere über die Probenkammer fluidisch verbunden sein. Wenn Teilbereiche unmittelbar fluidisch verbunden sind, sind insbesondere keine anderen Teilbereiche des Fluidsystems zwischen ihnen keine anderen Teilbereiche des Fluidsystems angeordnet sind. Eine direkte fluidische Verbindung schließt aber nicht aus, dass eines der Sperrelemente zwischen den unmittelbar fluidisch verbundenen Teilbereichen angeordnet ist.

Das Fluidsystem kann eine oder mehrere Kammern und/oder einen oder mehrere Kanäle umfassen, die jeweils zum Speichern und/oder zum Transport von Flüssigkeit ausgebildet sind und die mit der Probenkammer direkt oder indirekt fluidisch verbunden sind. Das Fluidsystem kann vollständig im Substrat ausgebildet sein oder Teilbereiche aufweisen, die im Substrat ausgebildet sind, und Teilbereiche, die in Aufnahmen im Substrat angeordnet sind. Solche Teilbereiche können beispielsweise in Form von Elementen wie Schläuchen oder Beuteln ausgebildet sein.

Das Fluidsystem kann Bereiche aufweisen, in denen der Boden und/oder die Wände eine Oberflächenfunktionalisierung, beispielsweise eine flächige oder eine strukturierte, insbesondere mikrostrukturierte, Oberflächenfunktionalisierung aufweisen. Alternativ oder zusätzlich kann das Fluidsystem Bereiche aufweisen, in denen der Boden strukturiert ist.

Das Fluidsystem kann insbesondere mehrere Fluidkanäle umfassen, wobei sich Fluidkanäle verzweigen und/oder Fluidkanäle zusammenlaufen. Fluidkanäle können beispielsweise einen rechteckigen Querschnitt aufweisen, insbesondere mit einer Breite von 0,3 mm bis 40 mm und/oder einer Länge von 3 mm bis 120 mm. Der Kanal kann eine Höhe von 0,04 mm bis 10 mm haben.

Das Fluidsystem kann als rein zirkuläres Fluidsystem oder als zirkuläres Fluidsystem mit Flüssigkeitsaustausch ausgebildet sein. Ein zirkuläres Fluidsystem ist derart ausgebildet, dass im bestimmungsgemäßen Gebrauch die Flüssigkeit mehrfach durch zumindest eine fluidische Struktur des Fluidsystems geleitet wird, wie dies typischerweise in einem Kreislauf der Fall ist. Dabei kann es sich z.B. um einen geschlossenen Kreislauf handeln, oder um ein System mit Flüssigkeitsreservoiren, die im bestimmungsgemäßen Gebrauch z.B. abwechselnd Flüssigkeiten aufnehmen und abgeben können, wobei die Flüssigkeit so geleitet wird, dass sie wiederholt die gleichen fluidischen Strukturen durchfließt.

Die Probenkammer dient im bestimmungsgemäßen Gebrauch als Untersuchungskammer oder Beobachtungskammer, beispielsweise für mikroskopische Untersuchungen, die insbesondere von unten durch den Boden der Probenkammer erfolgen können. In die Probenkammer können Zellen eingebracht werden und mikroskopisch untersucht werden. Alternativ oder zusätzlich können in der Probenkammer chemische Reaktionen beobachtet werden, die beispielsweise mittels fluoreszierenden Sonden sichtbar gemacht werden, beispielsweise durch Anbindung der Sonden auf einer Oberfläche immobilisierter chemischen Gruppen. Die Probenkammer kann auch zum Lagern der Proben ausgebildet sein, insbesondere gegenüber der Umgebung isolierbar ausgebildet sein.

Die Probenkammer kann eine im bestimmungsgemäßen Gebrauch verschlossene Öffnung aufweisen, die derart ausgebildet ist, dass eine Probe unmittelbar von außen in die Probenkammer eingebracht werden kann. Die Öffnung kann insbesondere an der Oberseite des Substrats unmittelbar oberhalb der Probenkammer angeordnet sein. Sie kann im bestimmungsgemäßen Gebrauch beispielsweise durch einen Stopfen, ein Kunststoffplättchen oder ein Glasplättchen verschlossen sein, wobei das Plättchen insbesondere mittels Doppelklebeband fixiert sein kann.

Das Substrat kann eine in der Probenkammer horizontal angeordnete poröse Membran umfassen, die die Probenkammer in einen unteren und einen oberen Bereich unterteilt.

Das Substrat kann Strukturen, die in der Probenkammer angeordnet und zur dreidimensionalen Kultivierung von Zellen ausgebildete sind, umfassen, insbesondere Hydrogelstrukturen oder sonstige 3D-ZellkulturTrägermaterialien (englisch "scaffolds"), beispielsweise aus porösen Materialien. Die Strukturen können die Probenkammer nur teilweise füllen, so dass in der Probenkammer Bereiche frei bleiben, durch die Flüssigkeit an der Hyrogelstruktur entlang strömen kann. Insbesondere kann die Probenkammer mindestens zwei solche Bereiche aufweisen, die durch die Hydrogelstruktur voneinander getrennt sind. Das Substrat kann insbesondere Hydrogel umfassen, das in der Probenkammer angeordnet ist und Bindestellen für Zellen oder Moleküle aufweist.

Die Probenkammer kann quaderförmig ausgebildet sein. Die Probenkammer kann ein oder mehrere Töpfchen aufweisen, die derart ausgebildet sind, dass ihre Form ermöglicht Sphäroide oder Organoide zu halten. Das Substrat kann in Form einer Multititerplatte ausgebildet sein.

Das Substrat kann zwei, insbesondere flüssigkeitsdicht, miteinander verbundene Platten, beispielsweise aus Glas oder Kunststoff, umfassen. Beide Platten können Spritzgussteile sein oder eine der Platten kann eine tiefgezogene und die andere eine ebene Folie sein oder eine Platte kann Spritzgussteil und die andere Platte eine ebene oder tiefgezogene Folie sein. Die Bodenplatte kann zwischen 0.2 µm und 10 mm dick sein. Die Platten können die gleiche Breite und Länge haben. Die Platten können irreversibel miteinander verbunden sein, insbesondere verklebt, Ultraschall-gebondet oder über Hitze oder ein Lösungsmittel gebondet sein.

Das Substrat kann aus Glas oder aus einem, insbesondere biokompatiblen, Kunststoff bestehen. Das Kunststoffmaterial kann beispielsweise Polycarbonat, Polystyrol, Polyethylen, Polyvinylchlorid, Cyclo-olefin Copolymer, Cyclo-olefin Polymer oder Polymethylmethacrylat umfassen.

Zumindest ein Teil des Substrats, insbesondere der Boden der Probenkammer, kann aus Material bestehen, das derart ausgebildet ist, dass eine mikroskopische Untersuchung der Probenkammer möglich ist, insbesondere durch den Boden der Probenkammer. Insbesondere kann die Doppelbrechung des Materials so gering sein, dass die mikroskopische Untersuchung möglich ist und/oder die Fluoreszenz des Materials kann so gering sein, dass eine Untersuchung mit Fluoreszenzmikroskopie möglich ist. Insbesondere kann der Brechungsindex des Materials zwischen 1,2 und 1,6 liegen.

Das Flüssigkeitsreservoir kann, wie oben erwähnt, ganz oder teilweise im Substrat ausgebildet sein. Alternativ kann zumindest ein Teilbereich des Flüssigkeitsreservoirs in einer Aufnahme im Substrat, beispielsweise in einer Aussparung, angeordnet sein. Das Flüssigkeitsreservoir kann mehrere Teilbereiche umfassen, wobei alle oder nur manche der Teilbereiche im Substrat ausgebildet sein können, oder keiner der Teilbereiche im Substrat ausgebildet ist.

Das Flüssigkeitsreservoir umfasst mindestens einen Teilbereich, der derart ausgebildet und im bestimmungsgemäßen Gebrauch derart angeordnet ist, dass darin Flüssigkeit aufbewahrt und/oder transportiert werden kann.

Das Flüssigkeitsreservoir kann mindestens eine Kammer und/oder mindestens einen Fluidkanal umfassen. Der Fluidkanal und/oder die Kammer können im Substrat ausgebildet sein. Alternativ kann beispielsweise der Fluidkanal im Substrat ausgebildet sein und die Kammer in Form eines in einer Aufnahme, beispielsweise einer Aussparung, im Substrat angeordneten Behältnisses, beispielsweise eines Beutels, ausgebildet sein. Das Behältnis kann in diesem Fall an den Fluidkanal angeschlossen sein, insbesondere an dessen Mündung eingesteckt, eingegossen, eingeschweißt oder eingeklebt sein.

Das Flüssigkeitsreservoir kann eine im Substrat ausgebildeten Kammer und einen Fluidkanal umfassen, der im unteren Bereich der Kammer in die Kammer mündet. Beispielsweise kann der Fluidkanal über eine Öffnung im Boden der Kammer oder in der Seitenwand der Kammer in die Kammer münden. Die Kammer kann sich nach unten hin verjüngen, insbesondere zu der Öffnung hin. Die Kammer kann also trichterförmig ausgebildet sein.

Das Flüssigkeitsreservoir kann im bestimmungsgemäßen Gebrauch zum Aufbewahren von Spülflüssigkeiten und/oder Prozessflüssigkeiten, insbesondere frischen bzw. noch nicht verwendeten Prozessflüssigkeiten, und/oder als Auffangreservoir für verbrauchte Flüssigkeiten oder Abfälle dienen. Das Flüssigkeitsreservoir kann im bestimmungsgemäßen Gebrauch verschiedene Funktionen haben, insbesondere mehrfach gefüllt und/oder entleert werden. Insbesondere kann ein Flüssigkeitsreservoir im bestimmungsgemäßen Gebrauch zum Aufbewahren einer frischen Spülflüssigkeit oder Prozessflüssigkeit dienen und als Abfallreservoir dienen nachdem die frische Spülflüssigkeit oder Prozessflüssigkeit aus dem Flüssigkeitsreservoir entnommen wurde.

Das Substrat kann derart ausgebildet sein, dass zumindest ein Teilbereich des Flüssigkeitsreservoirs zur Aufbewahrung und Untersuchung von Proben, insbesondere Zellen oder Molekülen, ausgebildet ist. Das Flüssigkeitsreservoir kann dann im bestimmungsgemäßen Gebrauch zum Aufbewahren und Untersuchen von Proben dienen, also als zusätzliche Probenkammer.

Das Sperrelement hat eine Sperrstellung und eine Durchlassstellung. Das Sperrelement kann derart ausgebildet sein, dass es von einer Sperrstellung in eine Durchlassstellung und von einer Durchlassstellung in eine Sperrstellung gebracht werden kann. Das heißt, dass das Öffnen des Sperrelements reversibel ist. Anders als beispielsweise bei durchstechbaren Membranen kann das Sperrelement und somit auch das Flüssigkeitsreservoir mehrfach verwendet werden.

Das passive Sperrelement kann derart ausgebildet und angeordnet sein, dass in der Durchlassstellung Flüssigkeit von dem Flüssigkeitsreservoir in die Probenkammer und/oder von der Probenkammer in das Flüssigkeitsreservoir gelangen kann. Das Sperrelement kann derart ausgebildet sein, dass es eine oder mehrere Durchlassrichtungen hat, insbesondere zwei entgegengesetzte Durchlassrichtungen.

Wenn hier die Begriffe "sperren" oder "verhindern" verwendet werden, bezieht sich das auf die vorgesehene Funktion des Sperrelements. Das heißt, die Begriffe sind im Rahmen üblicher systembedingter Abweichungen, beispielsweise aufgrund von Fertigungstoleranzen, zu verstehen.

Das passive Sperrelement kann ein passives druckabhängiges Ventil umfassen. Das Ventil kann beispielsweise derart ausgebildet sein, dass es eine Sperrstellung einnimmt, wenn auf beiden Seiten des Ventils der gleiche Druck anliegt. Das Ventil kann derart ausgebildet sein, dass durch Anlegen von Druck auf der einen Seite des Ventils das Ventil in eine Sperrstellung gebracht wird und/oder die Verschlusswirkung des Ventils verstärkt wird. Das Ventil kann alternativ oder zusätzlich derart ausgebildet sein, dass das Ventil durch Anlegen von Druck auf der anderen Seite des Ventils eine Durchlassstellung einnimmt.

Insbesondere kann das Sperrelement ein Rückschlagventil, beispielsweise ein Umbrella-Valve (Schirmventil) oder ein Duckbill-Valve (Entenschnabelventil) oder ein Kugelrückschlagventil, umfassen. Ein Rückschlagventil öffnet nur in eine Richtung. Alternativ kann das Sperrelement ein Ventil umfassen, das in beide Richtungen öffnet, beispielsweise ein Kreuzschlitzventil oder ein X-Fragm-Valve. Das Sperrelement kann auch eine Kombination von Ventilen umfassen.

Ein Schirmventil hat den Vorteil einer geringen Einbautiefe und ist daher für kompakte Substrate, insbesondere bei einer geringen Substratdicke, besonders geeignet.

Das Substrat kann derart ausgebildet und das Sperrelement derart ausgebildet und angeordnet sein, dass Totvolumina, insbesondere vor und nach dem Sperrelement, vermieden werden.

Das passive Sperrelement kann derart ausgebildet sein, dass es abhängig von den Druckverhältnissen im Fluidsystem die Sperrstellung oder die Durchlassstellung einnimmt.

Das Sperrelement kann insbesondere abhängig von dem relativen Druck auf den beiden Seiten des Sperrelements, eine Sperrstellung oder eine Durchlassstellung einnehmen. Dass das Sperrelement die Sperrstellung oder die Durchlassstellung einnimmt, umfasst, dass es in der Sperrstellung oder in der Durchlassstellung bleibt, also geschlossen oder geöffnet bleibt, oder von der Sperrstellung in die Durchlassstellung oder von der Durchlassstellung in die Sperrstellung übergeht, also öffnet oder schließt.

Die Druckverhältnisse können dabei insbesondere dadurch eingestellt werden, dass an das Fluidsystem Druck angelegt wird, wie unten noch im Detail erläutert, beispielsweise mittels eines über geeignete Anschlüsse angeschlossenen Pumpensystems.

Das Substrat, insbesondere das Fluidsystem und die Sperrelemente können insbesondere derart ausgebildet sein, dass allein durch Anlegen von Druck an das Fluidsystem der Flüssigkeitstransport in dem Substrat gesteuert werden kann.

Der Vorteil der oben beschriebenen Sperrelemente in dem beanspruchten Substrat ist, dass sehr einfach durch Anlegen eines Drucks an das Fluidsystem, beispielsweise über ein Pumpensystem, Flüssigkeiten gezielt durch das Fluidsystem bewegt werden können, und zugleich verhindert werden kann, dass sich Flüssigkeiten mischen oder in Bereiche des Fluidsystems gelangen, wenn dies nicht vorgesehen ist. Da in der Regel für das Antreiben der Flüssigkeit ohnehin ein externes Pumpensystem vorgesehen ist, genügt es, dieses entsprechend anzusteuern oder, falls nötig, zu modifizieren. Im Substrat müssen keine aktiven Komponenten, wie ansteuerbare Ventile oder Pumpen, vorgesehen werden. Das ermöglicht niedrige Fertigungskosten für das Substrat und geringe Fehleranfälligkeit. Letzteres ergibt sich daraus, dass aktive Komponenten typischerweise eine höhere Fehleranfälligkeit als passive Komponenten haben und auch bei der Ansteuerung die Komplexität durch jede zusätzliche aktive Komponente erhöht wird.

Das Substrat kann derart ausgebildet sein, dass in dem Substrat keine aktiven Komponenten angeordnet sind, insbesondere keine aktiven Ventile und/oder Pumpen. Unter aktiven Komponenten werden hier Komponenten verstanden, die, beispielsweise durch einen, beispielsweise elektrischen oder pneumatischen, Antrieb, direkt eingestellt und/und oder betrieben werden. Der Verzicht auf aktive Komponenten bedeutet, wie oben schon erläutert, dass die Fertigungskosten des Substrats und die Fehleranfälligkeit gering sind.

Das Flüssigkeitsreservoir kann derart verschließbar sein, dass es keine direkte Verbindung nach außen hat, insbesondere nach außen komplett verschlossen ist. Alternativ oder zusätzlich kann die Probenkammer derart verschließbar sein, dass sie keine direkte Verbindung nach außen hat, insbesondere nach außen komplett verschlossen ist. Insbesondere kann das gesamte Fluidsystem derart verschließbar sein, dass es keine direkte Verbindung nach außen hat, insbesondere nach außen komplett verschlossen ist.

Dabei ist hier mit "nach außen komplett verschlossen" gemeint, dass kein unmittelbarer Gasaustausch mit der Umgebung des Substrats erfolgen kann. Das heißt, dass beispielsweise alle Öffnungen, insbesondere die Anschlüsse, mit einem Pumpensystem verbunden oder mit einem Deckel oder Stopfen geschlossen werden können. Das Fluidsystem kann so für den Teil des Verfahrens, bei dem Flüssigkeiten durch das System transportiert werden, so weit abgedichtet sein, dass die Druckverhältnisse im System vollständig mittels des angeschlossenen Pumpensystems vorgegeben werden können. Außerdem kann so eine Verunreinigung des Fluidsystems im Gebrauch verhindert werden.

Wenn das System entsprechend nach außen verschlossen wird, kann dadurch die Gefahr von Verunreinigungen reduziert werden. Außerdem kann das Substrat, wenn das Verschließen nach dem Einfüllen der für einen Prozess vorgesehenen Flüssigkeiten und Probe, einsatzbereit transportiert werden. Eine Verwendung des Substrats für komplexe Prozesse, die Transport mehrerer Flüssigkeiten durch die Probenkammer umfassen, bei gleichzeitigem Verschließen nach außen wird dadurch ermöglicht, dass das System fluidisch betrachtet in sich geschlossen verwendet werden kann. Dazu können die Proben und die benötigten Flüssigkeiten in verschiedene Bereiche des Fluidsystems, die zunächst durch passive Sperrelemente voneinander getrennt sind, eingefüllt werden, und dann sukzessive bestimmte Sperrelemente geöffnet und geschlossen werden, so dass die Flüssigkeiten innerhalb des Substrats so transportiert werden, wie der Prozess es vorsieht. Das Substrat kann mindestens zwei wie oben beschriebe passive Sperrelemente umfassen. Das Substrat kann insbesondere derart ausgebildet sein, dass die Sperrelemente unabhängig voneinander die Sperrstellung oder die Durchlassstellung einnehmen.

So können mehrere Bereiche des Fluidsystems selektiv fluidisch verbunden oder getrennt werden. Das erhöht die Flexibilität bei der Verwendung, insbesondere auch für komplexere Verfahren.

Das Fluidsystem kann mehrere mit der Probenkammer fluidisch verbundene Flüssigkeitsreservoire umfassen und das Substrat kann mehrere passive Sperrelemente umfassen, die derart ausgebildet und angeordnet sind, dass in der Sperrstellung der Sperrelemente jeweils ein Flüssigkeitsaustausch zwischen der Probenkammer und einem der Flüssigkeitsreservoire gesperrt ist und/oder ein Flüssigkeitsaustausch zwischen den Flüssigkeitsreservoiren gesperrt ist. Wenn das Substrat die mehreren passiven Sperrelemente umfasst, kann das Substrat insbesondere derart ausgebildet sein, dass die Sperrelemente unabhängig voneinander eine Sperrstellung oder eine Durchlassstellung einnehmen können. Die Flüssigkeitsreservoire können, wie oben bereits beschrieben, jeweils ganz oder teilweise in dem Substrat ausgebildet sein. Insbesondere kann in dem Fluidsystem zwischen jedem der Flüssigkeitsreservoire und der Probenkammer jeweils mindestens eines der passiven Sperrelemente angeordnet sein.

Beispielsweise kann das Substrat derart ausgebildet sein, dass aus verschiedenen Flüssigkeitsreservoiren separat, insbesondere nacheinander, Flüssigkeit der Probenkammer zugeführt werden kann und/oder Flüssigkeit aus der Probenkammer einem oder mehreren Flüssigkeitsreservoiren zugeführt werden kann, indem die Sperrelemente entsprechende Sperrstellungen und Durchlassstellungen einnehmen. Dies kann beispielsweise, wie unten im Detail erläutert, durch Anlegen entsprechender Drücke an das Fluidsystem erreicht werden.

Die Anordnung ermöglicht, dass in dem Substrat auch komplexere Prozesse mit mehreren Flüssigkeiten, beispielsweise Prozessflüssigkeiten und/oder Spülflüssigkeiten, durchgeführt werden können, ohne dass nach jedem Schritt die entsprechende Flüssigkeit komplett aus dem Fluidsystem entfernt werden muss, also kein vollständiger Austausch der Flüssigkeit im Fluidsystem erforderlich ist. Es wird auch ermöglicht, dass solche Prozesse nach dem initialen Einfüllen der erforderlichen Flüssigkeiten nicht mehr von außen mit Flüssigkeit versorgt werden müssen. Somit kann das Substrat als autonomes und in sich geschlossenes System, insbesondere auch für komplexere Prozesse, verwendet werden. Das ermöglicht Flexibilität bei der Benutzung, insbesondere auch eine portable Nutzung, und reduziert die Gefahr von Verunreinigungen durch von außen zugeführte Flüssigkeiten.

Das Fluidsystem kann einen ersten Fluidkanal, der Teil des Flüssigkeitsreservoirs oder mit diesem unmittelbar fluidisch verbunden ist, und einen zweiten Fluidkanal, der mit der Probenkammer unmittelbar fluidisch verbunden ist, umfassen. Dabei können der erste Fluidkanal unmittelbar an den zweiten Fluidkanal angrenzend und das Sperrelement zwischen dem ersten Fluidkanal und dem zweiten Fluidkanal angeordnet sein.

Das Fluidsystem kann also beispielsweise zwei aneinander angrenzende und durch das Sperrelement getrennte Fluidkanäle umfassen, nämlich den ersten Fluidkanal und den zweiten Fluidkanal, wobei er erste Fluidkanal Teil des Flüssigkeitsreservoirs ist oder mit diesem unmittelbar fluidisch verbunden ist und wobei der zweite Fluidkanal in die Probenkammer mündet. Insbesondere kann das Flüssigkeitsreservoir mindestens zwei Teilbereiche umfassen, von denen ein Teilbereich in Form einer Kammer und der andere Teilbereich in Form des ersten Fluidkanals ausgebildet ist, wobei der erste Fluidkanal an einem Ende in die Kammer mündet und an einer anderen Stelle, insbesondere am anderen Ende, an den zweiten Fluidkanal angrenzt. Optional können einer, mehrere oder alle der Fluidkanäle im Substrat ausgebildet sein.

Insbesondere kann das Fluidsystem mindestens eine erste Probenkammer und eine zweite Probenkammer und mindestens zwei zweite Fluidkanäle umfassen, wobei der erste Fluidkanal über einen der zweiten Fluidkanäle mit der ersten Probenkammer und über einen anderen der zweiten Fluidkanäle mit der zweiten Probenkammer verbunden ist.

Die erste Probenkammer und die zweite Probenkammer können dabei jeweils über einen weiteren zweiten Fluidkanal und ein weiteres passives Sperrelement mit einem weiteren Flüssigkeitsreservoir fluidisch verbunden sein, wobei die beiden weiteren Flüssigkeitsreservoire derart ausgebildet sind, dass sie unabhängig voneinander mit Druck beaufschlagt werden können.

So können Prozesse beispielsweise an mehreren Probenkammern unter sehr vergleichbaren Bedingungen durchgeführt und dabei eine kompakte und einfache Fluidstruktur verwendet werden.

Insbesondere kann wahlweise in mehreren Probenkammern Flüssigkeit aus denselben Flüssigkeitsreservoiren verwendet werden oder Flüssigkeit aus mehreren Probenkammern kann in dasselbe Flüssigkeitsreservoir, insbesondere ein Abfallreservoir, abgeführt werden.

Einer der zweiten Fluidkanäle kann in die erste Probenkammer münden und der andere der zweiten Fluidkanäle kann in die zweite Probenkammer münden. Der erste Fluidkanal grenzt an beide zweiten Fluidkanäle an und ist von jedem der Fluidkanäle jeweils durch ein passives Sperrelement getrennt. Optional kann das Flüssigkeitsreservoir mindestens zwei Teilbereiche umfassen, von denen ein Teilbereich in Form einer Kammer und der andere Teilbereich in Form des ersten Fluidkanals ausgebildet ist, wobei der erste Fluidkanal an einem Ende in die Kammer mündet und an anderen Stellen an die zweiten Fluidkanäle angrenzt. Insbesondere kann der erste Fluidkanal an seinem anderen Ende an einen der zweiten Fluidkanäle angrenzen.

Das Fluidsystem kann mindestens ein erstes Sperrelement und ein zweites Sperrelement, mindestens ein erstes Flüssigkeitsreservoir und ein zweites Flüssigkeitsreservoir, mindestens zwei erste Fluidkanäle und einen zweiten Fluidkanal, der mit der Probenkammer unmittelbar fluidisch verbunden ist, umfassen. Einer der ersten Fluidkanäle kann Teil des ersten Flüssigkeitsreservoirs oder mit diesem unmittelbar fluidisch verbunden sein, ein anderer der ersten Fluidkanäle kann Teil des zweiten Flüssigkeitsreservoirs oder mit diesem unmittelbar fluidisch verbunden sein. Die ersten Fluidkanäle können unmittelbar an den zweiten Fluidkanal angrenzen und das erste Sperrelement kann zwischen dem einen der ersten Fluidkanäle und dem zweiten Fluidkanal angeordnet sein und das zweite Sperrelement kann zwischen dem anderen der ersten Fluidkanäle und dem zweiten Fluidkanal angeordnet sein.

Auf diese Weise können über den zweiten Fluidkanal Flüssigkeiten aus mehreren verschiedenen Flüssigkeitsreservoirs wahlweise gleichzeitig oder separat, insbesondere nacheinander, der Probenkammer zugeführt und/oder Flüssigkeiten aus der Probenkammer wahlweise separat dem ersten Flüssigkeitsreservoir oder dem zweiten Flüssigkeitsreservoir oder gleichzeitig beiden Flüssigkeitsreservoiren zugeführt werden.

Das ermöglicht eine kompakte und einfache Fluidstruktur, die dennoch auch für komplexere Verfahren verwendet werden kann.

Zumindest ein Teilbereich, insbesondere alle Teilbereiche, des einen der ersten Fluidkanäle kann unterhalb oder oberhalb des anderen der ersten Fluidkanäle angeordnet sein. Insbesondere kann der eine der ersten Fluidkanäle zumindest, insbesondere nur, in dem Teilbereich, in dem der eine der ersten Fluidkanäle unmittelbar an den zweiten Fluidkanal angrenzt, auf einer Höhe mit dem anderen der ersten Fluidkanäle angeordnet sein. Alternativ können die ersten Fluidkanäle auf einer Höhe, insbesondere nebeneinander, angeordnet sein.

Das Fluidsystem kann mindestens eine erste Probenkammer und eine zweite Probenkammer und mindestens zwei zweite Fluidkanäle umfassen, wobei mindestens einer der ersten Fluidkanäle über einen der zweiten Fluidkanäle mit der ersten Probenkammer und über einen anderen der zweiten Fluidkanäle mit der zweiten Probenkammer verbunden ist. Diesbezüglich gelten die oben bezüglich eines Substrats mit mindestens einer ersten und zweiten Probenkammer genannten Merkmale und Vorteile analog.

Das Fluidsystem kann mindestens ein erstes Sperrelement und ein zweites Sperrelement, mindestens ein erstes Flüssigkeitsreservoir und ein zweites Flüssigkeitsreservoir, mindestens zwei erste Fluidkanäle und mindestens zwei zweite Fluidkanäle, die mit der Probenkammer unmittelbar fluidisch verbunden sind, umfassen. Einer der ersten Fluidkanäle kann Teil des ersten Flüssigkeitsreservoirs oder mit diesem unmittelbar fluidisch verbunden sein und unmittelbar an einen der zweiten Fluidkanäle angrenzen, wobei dazwischen das erste Sperrelement angeordnet ist. Ein anderer der ersten Fluidkanäle kann Teil des zweiten Flüssigkeitsreservoirs oder mit diesem unmittelbar fluidisch verbunden sein und unmittelbar an einen anderen der zweiten Fluidkanäle angrenzen, wobei dazwischen das zweite Sperrelement angeordnet ist.

Optional kann das erste Sperrelement nur eine Durchlassrichtung von dem ersten Flüssigkeitsreservoir in die Probenkammer und das zweite Sperrelement nur eine Durchlassrichtung aus der Probenkammer in Richtung des zweiten Flüssigkeitsreservoirs haben.

Alternativ oder zusätzlich kann das erste Sperrelement insbesondere oberhalb des an das erste Sperrelement angrenzenden ersten Fluidkanals und unterhalb des an das erste Sperrelement angrenzenden zweiten Fluidkanals angeordnet sein. Alternativ oder zusätzlich kann das zweite Sperrelement unterhalb des an das zweite Sperrelement angrenzenden ersten Fluidkanals und oberhalb des an das zweite Sperrelement angrenzenden zweiten Fluidkanals angeordnet sein.

Wenn das Fluidsystem, beispielsweise wie oben beschrieben, einen ersten Fluidkanal und einen zweiten Fluidkanal umfasst, an den der erste Fluidkanal unmittelbar angrenzt, können der erste Fluidkanal und der zweite Fluidkanal, in einem Bereich, in dem der erste Fluidkanal unmittelbar an den zweiten Fluidkanal angrenzt, in Draufsicht auf das Substrat einander überlappend angeordnet sein und der erste Fluidkanal kann in diesem Bereich oberhalb oder unterhalb des zweiten Fluidkanals angeordnet sein. Das Sperrelement kann bei dieser Anordnung insbesondere ein Schirmventil sein. Das Sperrelement kann in Draufsicht mit dem ersten Fluidkanal und dem zweiten Fluidkanal überlappend angeordnet sein.

Optional kann der gesamte erste Fluidkanal oberhalb oder unterhalb des zweiten Fluidkanals angeordnet sein. Alternativ kann der erste Fluidkanal und/oder der zweite Fluidkanal derart geneigt und/oder verwinkelt ausgebildet sein, dass nur ein oder mehrere Teilbereiche des ersten Fluidkanals oberhalb bzw. unterhalb des zweiten Fluidkanals angeordnet sind, insbesondere zumindest ein Teilbereich, der unmittelbar an das Sperrelement angrenzt. Insbesondere kann der erste Fluidkanal und/oder der zweite Fluidkanal verwinkelt ausgebildet sein.

Eine Anordnung auf mehreren Ebenen mit überlappenden Fluidstrukturen ermöglicht eine kompakte Ausgestaltung des Substrats.

Ein wesentlicher Vorteil der Anordnung eines Sperrelements, beispielsweise eines Sperrventils, zwischen einem unteren und einem oberen Kanal, so dass die Fließrichtung vertikal ist, ist, dass das Sperrelement, insbesondere wenn es sich um ein Schirmventil handelt, einfach und mit relativ geringen Anforderungen an Passungen oder mechanische Präzision einzubauen sind.

Das Sperrelement derart ausgebildet und angeordnet sein, dass auch bei Fluktuationen eines mittels Pumpen angelegten Drucks, insbesondere auch bei Wegfall des angelegten Drucks, das Sperrelement zuverlässig die Sperrstellung einnimmt. So kann das Risiko einer ungewollten Vermischung mehrerer Flüssigkeiten im Substrat reduziert werden.

Das Substrat kann insbesondere mindestens zwei erste Fluidkanäle und zwei zweite Fluidkanäle aufweisen, wobei je einer der ersten Fluidkanäle an je einen der zweiten Fluidkanäle angrenzt und wobei einer der ersten Fluidkanäle unterhalb des daran angrenzenden zweiten Fluidkanals angeordnet ist und der andere der ersten Fluidkanäle oberhalb des daran angrenzenden Fluidkanals angeordnet ist.

Insbesondere kann die vorgesehene Transportrichtung der Flüssigkeit von dem ersten Flüssigkeitsreservoir durch den einen der ersten Fluidkanäle und den einen der zweiten Fluidkanäle in die Probenkammer hinein und durch den anderen der zweiten Fluidkanäle und den anderen der zweiten Fluidkanäle aus der Kammer heraus sein. Die Flüssigkeit muss dann jeweils nach oben, also je nach Anwendungsfall gegebenenfalls gegen die Schwerkraft, durch das jeweilige Sperrelement strömen. So kann die Schwerkraft genutzt werden, um das Risiko einer ungewollten Vermischung mehrerer Flüssigkeiten im Substrat zu reduzieren.

Das Substrat kann mindestens einen Anschluss, beispielsweise für ein Pumpensystem, aufweisen, wobei das Substrat derart ausgebildet ist, dass über den Anschluss Gas in das Flüssigkeitsreservoir bzw. genau eines der Flüssigkeitsreservoire oder in zwei oder mehr der Flüssigkeitsreservoire eintreten, insbesondere gepumpt werden, kann. Alternativ oder zusätzlich kann das Substrat mindestens einen ersten Anschluss und einen zweiten Anschluss, jeweils beispielsweise für das Pumpensystem, aufweisen, wobei das Substrat derart ausgebildet ist, dass über den ersten Anschluss Gas in eines der Flüssigkeitsreservoire eintreten, insbesondere gepumpt werden, kann und über den zweiten Anschluss Gas in ein anderes der Flüssigkeitsreservoire eintreten, insbesondere gepumpt werden, kann.

Durch das Pumpen von Gas in das Flüssigkeitsreservoir werden die Druckverhältnisse im Substrat verändert, insbesondere eingestellt. So kann gezielt bewirkt werden, welche der Sperrelemente eine Sperrstellung und welche der Sperrelemente eine Durchlassstellung einnehmen. Zudem kann optional durch das Pumpen gezielt bewirkt werden, in welche Richtung das bzw. die Sperrelemente Flüssigkeit durchlassen, also die Durchlassrichtung eingestellt werden.

Das Sperrelement bzw. die Sperrelemente können durch eine Minimaldruckdifferenz charakterisiert sein, die über dem jeweiligen Sperrelement anliegen muss, um das Sperrelement zu öffnen, die sogenannte Öffnungsdruckdifferenz oder kurz der Öffnungsdruck.

Beispielsweise kann dann im bestimmungsgemäßen Gebrauch durch das Erhöhen des Drucks in einem Flüssigkeitsreservoir und Erniedrigen des Drucks in einem zweiten Flüssigkeitsreservoir eine Druckdifferenz über einem zwischen den zwei Flüssigkeitsreservoiren angeordneten Sperrelement aufgebaut werden, die größer als die Öffnungsdruckdifferenz ist. Dabei kann der Überdruck in dem einen Flüssigkeitsreservoir so niedrig, und der Unterdruck im zweiten Flüssigkeitsreservoir so niedrig gewählt werden, dass alleine der jeweilige Über- bzw. Unterdruck nicht ausreicht, um das Sperrelement zu öffnen. Dadurch bleiben alle im System befindlichen Sperrelemente an denen jeweils entweder nur der Überdruck oder nur der Unterdruck anliegt, gesperrt.

Der Anschluss kann insbesondere als Öffnung in einer der Außenwände des Flüssigkeitsreservoirs oder in einem Deckel oder Stopfen, mit dem das Flüssigkeitsreservoir verschlossen ist, ausgebildet sein.

In dieser Öffnung kann beispielsweise unmittelbar ein Schlauch des Pumpensystems angeschlossen werden, beispielsweise durch Einstecken des Schlauchs in die Öffnung, oder über die Öffnung kann eine indirekte Verbindung mit dem Schlauch des Pumpensystems hergestellt werden, beispielsweise über einen Adapter oder auf das Substrat aufgesetztes separates Element, wie beispielsweise ein auf dem Substrat befestigtes (unter Umständen nicht zum Substrat gehörendes) Flüssigkeitsreservoir.

Deckel oder Stopfen können insbesondere zumindest im Bereich der Öffnung aus einem weichen Material, beispielsweise Polyethylen oder Silikon, das durch mechanische Presspassung zu einer Abdichtung führt, wenn der Schlauch in die Öffnung gesteckt ist, ausgebildet sein. Insbesondere können mehrere Deckel oder Stopfen auch in einer Komponente zusammengefasst werden, die derart ausgebildet ist, dass ermöglicht wird, durch einmaliges Aufstecken mehrere, insbesondere alle, vorgesehenen Verbindungen zum Pumpensystem zugleich herzustellen. Optional können in einem Deckel oder Stopfen jeweils mehrere Öffnungen für Druckleitungen sein, beispielsweise eine, die im ordnungsgemäßen Betrieb für das Liefern von Überdruck und eine die im ordnungsgemäßen Betrieb für das Liefern von Unterdruck vorgesehen ist. Das Substrat kann ein Dichtelement umfassen, das derart ausgebildet und angeordnet ist, dass der Deckel oder Stopfen abgedichtet wird, beispielsweise in Form von Dichtmasse oder Dichtbändern oder in Form eines O-Rings.

Das Substrat kann mindestens einen Filter, beispielsweise einen Sterilfilter, umfassen, der derart ausgebildet und angeordnet ist, dass in das Flüssigkeitsreservoir einströmendes Gas den Filter passiert. Der Filter kann derart ausgebildet sein, dass das einströmende Gas gefiltert wird, insbesondere, dass Mikroben aus Gas herausgefiltert werden. Insbesondere kann der mindestens eine Anschluss einen Filter umfassen und/oder ein Filter kann an dem Anschluss angebracht sein. So kann das Risiko, dass Verunreinigungen in das Fluidsystem, insbesondere in die Probenkammer, gelangen, reduziert werden.

An die Flüssigkeitsreservoire können Sterilfilter fest montiert sein z.B. mit Luerlockverbindung. D.h., dass das Substrat aufgrund der Sterilfilter auch in unsteriler Umgebung transportiert werden kann, ohne das Substratinnere unsteril zu machen. An die Sterilfilter können dann Gasschläuche, beispielsweise die unten im Zusammenhang mit dem System beschriebenen Schläuche, angeschlossen und die Reservoire mit Druck beaufschlagt werden. Auch in diesem Fall schützen die Sterilfilter das Substratinnere vor Verunreinigung.

Das Substrat kann optional ein Verdrängungselement umfassend eine Kammer, die keine fluidische Verbindung mit dem Flüssigkeitsreservoir hat, und mindestens einen Anschluss, beispielsweise für ein bzw. das Pumpensystem, aufweisen, wobei das Verdrängungselement derart ausgebildet ist, dass durch Pumpen von Gas und/oder Flüssigkeit in die Kammer das Volumen der Kammer erhöht wird, und wobei das Substrat derart ausgebildet ist, dass über den Anschluss Gas und/oder Flüssigkeit in die Kammer des Verdrängungselements gepumpt werden kann und dass das Volumen des Flüssigkeitsreservoirs reduziert wird, wenn durch das Pumpen von Gas und/oder Flüssigkeit in die Kammer das Volumen der Kammer erhöht wird.

Dazu kann die Kammer des Verdrängungselements mindestens eine Außenwand aufweisen, die durch das Pumpen von Gas und/oder Flüssigkeit in die Kammer verformbar, insbesondere dehnbar, ist. Insbesondere kann das Verdrängungselement in Form eines Beutels ausgebildet sein, der bei Zufuhr von Gas und/oder Flüssigkeit aufgeblasen wird. Der Beutel kann eine dehnbare Außenwand haben, insbesondere in Form eines Ballons ausgebildet sein.

Das Substrat kann derart ausgebildet sein, dass in die Kammer des Verdrängungselements einströmendes Gas keinen Filter passiert.

Das Verdrängungselement ermöglicht, dass über den Anschluss kein Gas von außen in das Flüssigkeitsreservoir und somit das Fluidsystem eintritt und dennoch mittels einer Pumpe der Druck im Fluidsystem verändert werden kann. So kann, insbesondere auch ohne den Einsatz eines Filters, das Risiko, dass Verunreinigungen in das Fluidsystem gelangen, reduziert werden.

Die Kammer des Verdrängungselements kann in dem Flüssigkeitsreservoir oder an das Flüssigkeitsreservoir angrenzend in einer Aufnahme im Substrat angeordnet sein. Dadurch, dass sich die Kammer des Verdrängungselements und das Flüssigkeitsreservoir ein begrenztes Volumen teilen, wird dann durch Vergrößern des Volumens der Kammer das effektive Volumen des Flüssigkeitsreservoirs reduziert. Die Kammer des Verdrängungselements kann alternativ oder zusätzlich derart ausgebildet und angeordnet sein, dass eine Erhöhung des Volumens der Kammer eine bewegliche Komponente des Verdrängungselements derart bewegt, dass das Volumen des Flüssigkeitsreservoirs reduziert wird.

Das Substrat kann zwei Verdrängungselemente in Form von Beuteln umfassen, wobei der eine Beutel im bestimmungsgemäßen Gebrauch zunächst frisches Medium enthält und der zweite Beutel als Auffangreservoir für verbrauchte Flüssigkeit dient. Beispielsweise können im Gebrauch in der Ausgangssituation beide Beutel eine Aussparung im Substrat vollständig ausfüllen. Transportiert man nun das frische Medium aus dem einen Beutel, beispielsweise mittels einer Membranpumpe oder Schlauchquetschpumpe, durch das Fluidsystem in den zweiten Beutel, der als Auffangreservoir dient, bleibt das Gesamtvolumen in der Aussparung konstant. Diese Anordnung ist eine sehr platzsparende Variante der Fluidspeicherung.

Insbesondere können alle Anschlüsse des Substrats jeweils ausschließlich mit einer Kammer eines solchen Verdrängungselements verbunden sein. Somit tritt über keinen der Anschlüsse Gas von außen in das Fluidsystem ein. Insbesondere kann das Fluidsystem nach außen komplett verschlossen sein. Das Fluidsystem kann also vollständig von der Umgebung isoliert werden. So kann, insbesondere auch ohne den Einsatz von Filtern, das Risiko, dass Verunreinigungen in das Fluidsystem gelangen, reduziert werden.

Die Erfindung betrifft auch ein System umfassend eines der oben beschriebenen Substrate und ein Pumpensystem, das zum Anschließen an das Substrat, insbesondere an einen oder mehrere der Anschlüsse des Substrats, ausgebildet ist. Insbesondere kann das Pumpensystem zum Anschließen an das Substrat, insbesondere das Fluidsystem, über den bzw. die oben beschriebenen Anschlüsse ausgebildet sein.

Das Pumpensystem kann mindestens eine Pumpe umfassen. Optional kann es auch mehrere Pumpen umfassen, insbesondere, wenn das Substrat mehrere Anschlüsse aufweist. Dann können in einer Anordnung zum bestimmungsgemäßen Gebrauch verschiedene Anschlüsse mit verschiedenen Pumpen verbunden sein. Alternativ oder zusätzlich kann das Pumpensystem mehrere Pumpen aufweisen, von denen im bestimmungsgemäßen Gebrauch mindestens eine Pumpe nur zum Anlegen von Unterdruck und eine andere Pumpe nur zum Anlegen von Überdruck verwendet wird.

Bei der Pumpe kann es sich um eine Luftdruckpumpe handeln, insbesondere eine Luftdruckpumpe, die ermöglicht, den Druck rückgekoppelt einzustellen, beispielsweise in einem Bereich zwischen -300 mbar und 300 mbar. Bei einer Luftdruckpumpe ist das Antriebsfluid gasförmig, insbesondere kann es sich um Luft handeln. Die Pumpe in Form einer Schwingankerpumpe oder Membranpumpe ausgebildet sein. Alternativ kann die Pumpe in Form einer Kolbenpumpe, insbesondere in Form einer Spritzenpumpe, ausgebildet sein. Diese Art von Pumpen sind für eine präzise Kontrolle der beförderten Menge von Antriebsfluid besonders geeignet.

Alternativ zu einem gasförmigen Antriebsfluid kann ein flüssiges Antriebsfluid verwendet werden. Insbesondere kann als Antriebsfluid Öl, beispielsweise Mineralöl, Silikon oder eine andere hydrophobe Flüssigkeit, beispielsweise Fluorcarbon, verwendet werden. Insbesondere kann die Pumpe eine Kolbenpumpe sein. Öle und Wasser sind kaum komprimierbar, wodurch man hohe Genauigkeit hinsichtlich der mittels der Kolbenpumpe geförderten Volumina erzielen kann.

Das Pumpensystem kann ein oder mehrere Ventile und/oder ein oder mehrere Schläuche umfassen. Insbesondere kann das Pumpensystem Schläuche umfassen, mit denen es im bestimmungsgemäßen Gebrauch an das Substrat, insbesondere die Anschlüsse des Substrats, angeschlossen ist. das Pumpensystem kann Sterilfilter umfassen, die in oder an diesen Schläuchen derart angeordnet sind, dass das Antriebsfluid vor dem Eintritt in das Substrat gefiltert wird.

Insbesondere kann das Pumpensystem mindestens eine Pumpe und eine Ventilschaltung, die insbesondere derart ausgebildet ist, dass sie als Druckverteiler zu dem bzw. den Anschlüssen des Substrats dient, umfassen. Die Ventilschaltung kann aktive und/oder passive Ventile umfassen.

Die Ventilschaltung kann mindestens ein Mehrwegeventil umfassen, beispielsweise ein Drehventil mit drei Anschlüssen und drei Drehstellungen. Alternativ oder zusätzlich kann die Ventilschaltung einen y-Verbinder und zwei Schlauchquetschventile umfassen. Alternativ oder zusätzlich kann die Ventilschaltung ein oder mehrere elektrisch aktuierte Pneumatikventile umfassen, die beispielsweise derart ausgebildet sind, dass sie bei angelegter elektrischer Spannung offen und ohne angelegte elektrische Spannung geschlossen sind oder dass sie bei angelegter elektrischer Spannung geschlossen und ohne angelegte elektrische Spannung offen sind. Solche Pneumatikventile können auf einen Block, beispielsweise aus Aluminium, aufgebracht sein, wobei in dem Block Kanäle, beispielsweise in Form von Röhren, angeordnet sind, die an Anschlüssen für Luftdruckschläuche enden.

Die Ventilschaltung kann optional Mikroventile umfassen, die im bestimmungsgemäßen Gebrauch insbesondere auf dem Substrat befestigt sein können. Die Mikroventile können derart ausgebildet sein, dass sie das Antriebsfluid auf die Anschlüsse des Substrats verteilen und mittels Pneumatikventilen oder eines Kanalquetschmechanismus geschaltet werden können. Die Mikroventile können also als Pneumatikadapter dienen.

Bei dem oben genannten Drehventil mit drei Anschlüssen und drei Drehstellungen kann an einem Anschluss des Ventils die Pumpe angeschlossen sein und an den beiden anderen Anschlüssen des Drehventils können zwei Anschlüsse des Substrats angeschlossen sein. In einer ersten Drehstellung kann der Durchgang von der Pumpe unterbrochen sein, in einer zweiten Drehstellung kann die Pumpe nur mit einem der zwei Anschlüsse des Substrats verbunden sein und in der dritten Drehstellung kann die Pumpe nur mit dem anderen Anschluss des Substrats verbunden sein.

Bei der oben genannten Ventilschaltung mit einem y-Verbinder und zwei Schlauchquetschventilen (SQV) können über den y-Verbinder drei Schläuche miteinander verbunden sein, von denen ein erster Schlauch mit der Pumpe verbunden ist und der zweite und dritte Schlauch jeweils mit einem Anschluss des Substrats. Der zweite Schlauch verläuft durch ein erstes der Schlauchquetschventile und der dritte Schlauch verläuft durch ein zweites der Schlauchquetschventile. Wenn beide Schlauchquetschventile geschlossen sind, ist der Antriebsfluidfluss unterbrochen. Wenn eines der Schlauchquetschventile geöffnet und das andere geschlossen ist, wird nur zu einem der Anschlüsse des Substrats Antriebsfluid gepumpt. Wenn beide Schlauchquetschventile geöffnet sind, wird zu beiden Anschlüssen des Substrats Antriebsfluid gepumpt.

Das System kann mindestens einen Adapter, insbesondere auch eine Adapterleiste, umfassender bzw. die derart ausgebildet ist, über den bzw. die da Pumpensystem im bestimmungsgemäßen Gebrauch an das Substrat angeschlossen ist.

Das System kann optional eine Steuervorrichtung zum Ansteuern des Pumpensystems umfassen. Insbesondere kann die Steuervorrichtung zum Ansteuern einer oder mehrere Pumpen des Pumpensystems und/oder eines oder mehrerer Ventile des Pumpensystems ausgebildet sein.

Die Steuervorrichtung kann derart ausgebildet sein, dass sie das Pumpensystem, insbesondere eine oder mehrere Pumpen und/oder eines oder mehre Ventile des Pumpensystems, derart ansteuert, dass Druck an das Fluidsystem so angelegt wird, dass mindestens eines der Sperrelemente eine Durchlassstellung einnimmt und Flüssigkeit von einem der Flüssigkeitsreservoire in die Probenkammer hinein und/oder von der Probenkammer in eines der Flüssigkeitsreservoire transportiert wird, und optional derart, dass zugleich mindestens ein anderes der Sperrelemente eine Sperrstellung einnimmt und/oder ein anderes der Sperrelemente eine Durchlassstellung einnimmt.

Das System kann optional ein oder mehrere Flüssigkeitsreservoire umfassen, die im bestimmungsgemäßen Gebrauch auf dem Substrat befestigt sind und mit dem Fluidsystem fluidisch verbindbar sind.

Diese auf dem Substrat befestigten Flüssigkeitsreservoire können im bestimmungsgemäßen Gebrauch auf das Substrat gesteckt oder geschraubt sein. Das Substrat kann beispielsweise weibliche Luer- oder Luerlock-Adapter aufweisen, insbesondere an den oben beschriebenen Anschlüssen, und die befestigten Flüssigkeitsreservoire jeweils einen männlichen Luer- oder Luerlock-Adapter, der im bestimmungemäßen Gebrauch in die weiblichen Luer- oder Luerlock-Adapter gesteckt bzw. eingeschraubt wird. Der männliche Luer- oder Luerlock-Adapter kann an der Bodenseite des auf dem Substrat befestigten Flüssigkeitsreservoirs angeordnet sein. Das auf dem Substrat befestigte Flüssigkeitsreservoir kann in Form einer Spritze ausgebildet sein. Die im bestimmungsgemäßen Gebrauch auf dem Substrat befestigten Flüssigkeitsreservoire können mechanisch derart miteinander verbunden sein, dass die relative Anordnung der männlichen Luer- oder Luerlock-Adapter der relativen Anordnung der weiblichen Luer- oder Luerlock-Adapter entspricht. So können alle miteinander verbundenen Flüssigkeitsreservoire zugleich an dem Substrat befestigt werden.

Die Erfindung betrifft auch ein Verfahren zur Verwendung eines der oben beschriebenen Substrate oder Systeme. Das Verfahren umfasst ein Anlegen von Druck an das Fluidsystem, insbesondere mittels des Pumpensystems, derart, dass mindestens eines der Sperrelemente eine Durchlassstellung einnimmt und Flüssigkeit von einem der Flüssigkeitsreservoire in die Probenkammer hinein und/oder Flüssigkeit von der Probenkammer in eines der Flüssigkeitsreservoire transportiert wird, und optional derart, dass zugleich mindestens ein anderes der Sperrelemente eine Durchlassstellung einnimmt und/oder ein anderes der Sperrelemente eine Sperrstellung einnimmt.

Das Anlegen von Druck kann derart erfolgen, dass eine erste Flüssigkeit, beispielsweise eine Spülflüssigkeit oder ein Puffer, aus einem ersten Flüssigkeitsreservoir in die Probenkammer transportiert und dadurch eine zweite Flüssigkeit aus der Probenkammer verdrängt wird, dass die zweite Flüssigkeit in ein zweites Flüssigkeitsreservoir transportiert wird und anschließend eine dritte Flüssigkeit aus einem dritten Flüssigkeitsreservoir in die Probenkammer transportiert und dadurch die erste Flüssigkeit aus der Probenkammer verdrängt wird, insbesondere derart, dass die erste Flüssigkeit in das zweite Flüssigkeitsreservoir oder in ein viertes Flüssigkeitsreservoir transportiert wird. Das Anlegen von Druck mittels des Pumpensystems kann ein Betätigen der Pumpe oder Pumpen und/oder des Ventils oder der Ventile des Pumpensystems umfassen, insbesondere ein Ansteuern der Pumpe oder Pumpen und/oder des Ventils oder der Ventile des Pumpensystems, beispielsweise mittels einer Steuervorrichtung.

So erfolgt ein Austausch der Flüssigkeiten in der Probenkammer. Bei der zweiten und dritten Flüssigkeit kann es sich um Prozessflüssigkeiten handeln, die durch dieses Verfahren ausgetauscht werden. Wenn die erste Flüssigkeit eine Spülflüssigkeit oder ein Puffer ist, kann eine Vermischung der Prozessflüssigkeiten in der Probenkammer zuverlässig verhindert werden.

Das Verfahren kann insbesondere auch eine Untersuchung von Proben, insbesondere Zellen oder Molekülen, in der Probenkammer umfassen, insbesondere eine mikroskopische Untersuchung, beispielsweise durch den Boden der Probenkammer. Insbesondere eine Untersuchung vor, während und/oder nach dem Transport von Flüssigkeiten durch die Probenkammer.

Das Verfahren kann vor dem oben beschriebenen Transport von Flüssigkeiten und/oder vor der Untersuchung von Proben, ein Einfüllen je einer Flüssigkeit in das bzw. mindestens eines der Flüssigkeitsreservoire, insbesondere in mindestens zwei, insbesondere mindestens drei der Flüssigkeitsreservoire, umfassen. Dabei können das Sperrelement bzw. eines, mehrere oder alle der Sperrelemente in einer Sperrstellung sein.

Das Verfahren kann vor der Untersuchung von Proben, insbesondere vor dem oben beschriebenen Transport von Flüssigkeiten, ein Einbringen einer Probe, insbesondere ein Einbringen von Zellen oder Molekülen, in die Probenkammer umfassen. Dabei kann das Sperrelement bzw. eines, mehrere oder alle Sperrelemente in einer Sperrstellung sein.

Das Verfahren kann umfassen, dass vor dem oben beschriebenen Transport von Flüssigkeiten und/oder vor der Untersuchung von Proben ein bzw. das Pumpensystem an das Substrat, insbesondere an den oder die oben beschriebenen Anschlüsse angeschlossen wird.

Das Verfahren kann umfassen, dass vor dem oben beschriebenen Transport von Flüssigkeiten und/oder vor der Untersuchung von Proben ein Abdichten der Flüssigkeitsreservoire und/oder der Probenkammer, insbesondere ein Abdichten des Fluidsystems erfolgt, insbesondere nach dem Einfüllen der Flüssigkeiten und/oder der Probe.

Die Erfindung betrifft auch ein Verfahren zur Herstellung des Substrats.

Merkmale und Vorteile die oben im Zusammenhang mit dem Substrat beschrieben wurden, gelten für das System und das Verfahren analog.

Weitere Merkmale und Vorteile werden nachfolgend anhand der beispielhaften Figuren erläutert. Dabei zeigt:
Figuren 1a und 1b eine schematische, nicht maßstabsgetreue Draufsicht und Schnittansicht eines Substrats einer ersten Ausführungsform;
Figuren 2a bis 2c schematische, nicht maßstabsgetreue Schnittansichten eines Sperrelements in Form eines Rückschlagventils;
Figuren 3a bis 3c schematische, nicht maßstabsgetreue Schnittansichten eines Substrats im Bereich eines Flüssigkeitsreservoirs;
Figur 4 eine schematische, nicht maßstabsgetreue Schnittansicht eines Substrats einer zweiten Ausführungsform;
Figuren 5a bis 5c schematische, nicht maßstabsgetreue Darstellungen von Teilbereichen eines Substrats;
Figuren 6a bis 6c schematische, nicht maßstabsgetreue Darstellungen von Probenkammern;
Figuren 7a und 7b schematische, nicht maßstabsgetreue Darstellungen von möglichen Konfigurationen des Fluidsystems und der Sperrelemente;
Figur 8 schematische, nicht maßstabsgetreue Darstellung einer Ausführungsform eines Systems; und
Figuren 9a bis 9e schematische, nicht maßstabsgetreue Darstellungen von Ventilschaltungen.

Im Folgenden und in den Figuren werden in den verschiedenen Ausführungsbeispielen, sofern nicht anders spezifiziert die gleichen Bezugszeichen für gleiche oder entsprechende Elemente verwendet. Die unten genannten Schnittansichten sind jeweils Schnitte entlang der Längsachse des Substrats.

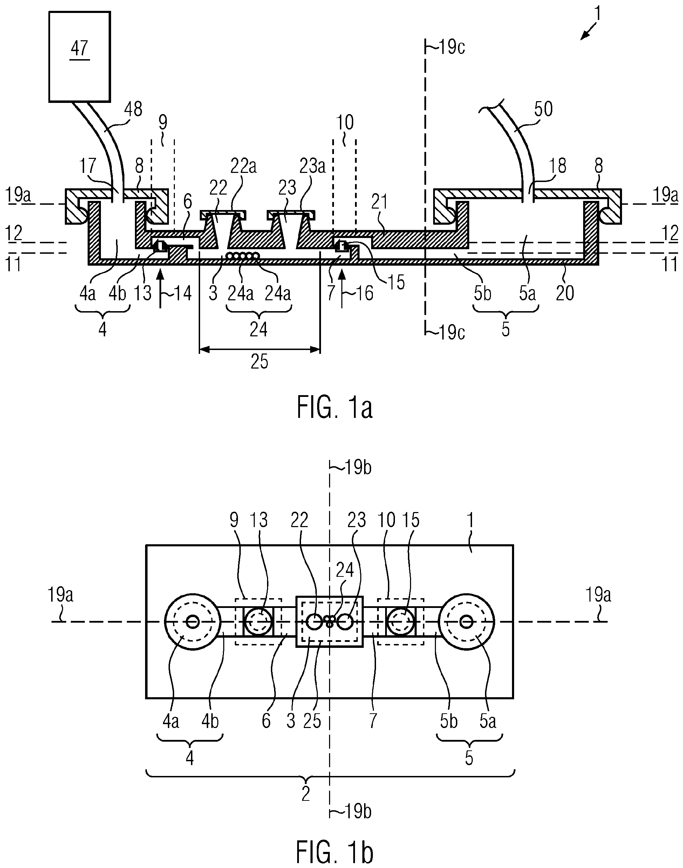

Figuren 1a und 1b zeigen eine erste Ausführungsform eines Substrats 1 zur Untersuchung von Proben. Das Substrat umfasst ein Fluidsystem 2.

Das Fluidsystem umfasst eine in dem Substrat ausgebildete Probenkammer 3 und ein mit der Probenkammer verbundenes erstes Flüssigkeitsreservoir 4, das eine Kammer 4a und einen Fluidkanal 4b umfasst. Weiterhin umfasst das Fluidsystem ein zweites Flüssigkeitsreservoir 5, das eine Kammer 5a und einen Fluidkanal 5b umfasst. In der vorliegenden Ausführungsform sind die Flüssigkeitsreservoire 4 und 5 vollständig in dem Substrat ausgebildet. Dies ist jedoch nicht zwingend der Fall. Beispielsweise kann optional zumindest eine der Kammern nicht in dem Substrat ausgebildet, sondern in einer Aufnahme im Substrat angeordnet sein, beispielsweise als Beutel, wie unten beschrieben.

Das Fluidsystem umfasst außerdem einen Fluidkanal 6, der die Probenkammer 3 und das Flüssigkeitsreservoir 4 fluidisch verbindet. Außerdem umfasst das Fluidsystem einen Fluidkanal 7, der die Probenkammer 3 und das Flüssigkeitsreservoir 5 fluidisch verbindet.

Der Fluidkanal 6 grenzt fluidisch unmittelbar an das erste Flüssigkeitsreservoir 4 an, in dieser Ausführungsform beispielsweise an den Fluidkanal 4b. Der Fluidkanal 7 grenzt unmittelbar an das zweite Flüssigkeitsreservoir 5 an, in dieser Ausführungsform beispielsweise an den Fluidkanal 5b.

In der in Figur 1b gezeigten Draufsicht ist gezeigt, dass der Fluidkanal 4b und der Fluidkanal 6 einander in dem Bereich 9, in dem sie unmittelbar aneinander angrenzen, überlappen und dass der Fluidkanal 5b und der Fluidkanal 7 einander in dem Bereich 10, in dem sie unmittelbar aneinander angrenzen, überlappen.

In dem in Figur 1a gezeigten Schnitt ist gezeigt, dass der Fluidkanal 4b in dem Bereich 9, in dem der Fluidkanal 4b und der Fluidkanal 6 einander überlappen, unterhalb des Fluidkanals 6 angeordnet ist. Weiterhin ist in dem in Figur 1a gezeigten Schnitt gezeigt, dass der Fluidkanal 5b in dem Bereich 10, in dem der Fluidkanal 5b und der Fluidkanal 7 einander überlappen, oberhalb des Fluidkanals 7 angeordnet ist.

Im vorliegenden Beispiel verlaufen die Fluidkanäle 4b, 6 und 7 jeweils horizontal. Der Fluidkanal 5b ist verwinkelt ausgebildet und umfasst zwei horizontal verlaufende Teilbereiche und einen vertikal verlaufenden Teilbereich, der die beiden horizontalen Teilbereiche verbindet. Einer der horizontalen Teilbereiche grenzt unmittelbar an den Fluidkanal 7 an, der andere der horizontalen Teilbereiche grenzt unmittelbar an die Kammer 5a an.

Beispielhaft sind der Fluidkanal 4b, der Fluidkanal 7 und einer der horizontalen Teilbereiche des Fluidkanals 5b, insbesondere der unmittelbar an die Kammer 5a angrenzende Teilbereich des Fluidkanals 5b, in einer ersten Ebene 11 angeordnet. Der Fluidkanal 6 und der ein anderer der horizontalen Teilbereiche des Fluidkanals 5b, insbesondere der unmittelbar an den Fluidkanal 7 angrenzende Teilbereich des Fluidkanals 5b, sind in einer zweiten Ebene 12 angeordnet. In diesem Beispiel ist die erste Ebene unterhalb der zweiten Ebene angeordnet.

Das Substrat umfasst weiterhin ein erstes passives Sperrelement 13, das zum Sperren eines Flüssigkeitsaustauschs zwischen der Probenkammer und dem ersten Flüssigkeitsreservoir 4 ausgebildet und angeordnet ist. Insbesondere ist das erste passive Sperrelement zwischen dem Fluidkanal 4b und dem Fluidkanal 6 angeordnet und grenzt unmittelbar an den Fluidkanal 4b und den Fluidkanal 6 an. Das Sperrelement 13 ist in dem Bereich 9 angeordnet, in dem der Fluidkanal 4b und der Fluidkanal 6 einander überlappen.

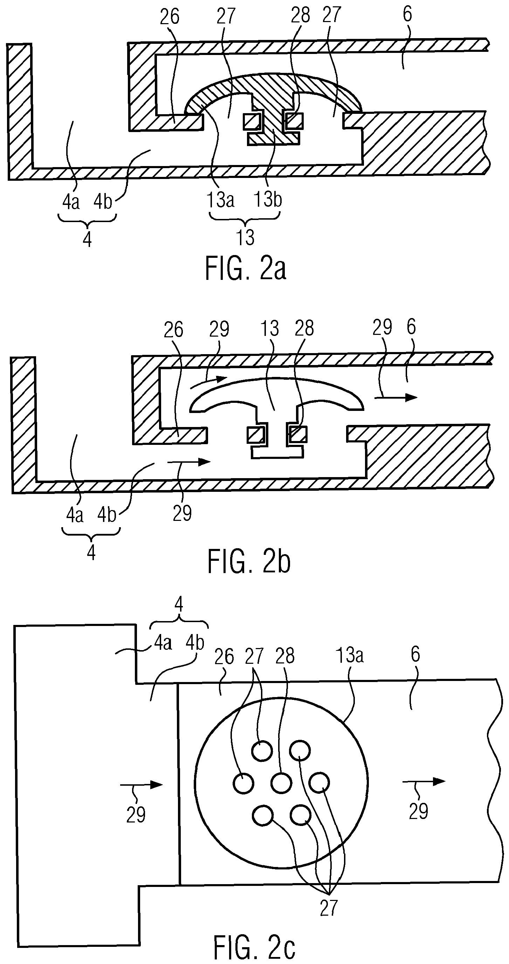

Das erste Sperrelement kann in Form eines Rückschlagventils, beispielsweise in Form eines Schirmventils, ausgebildet sein. Die Funktionsweise eines solchen Rückschlagventils wird im Zusammenhang mit Figuren 2a bis 2c noch näher erläutert. Alternativ zu einem Schirmventil kann ein Entenschnabelventil oder ein anderes Ventil verwendet werden. Das Sperrelement 13 kann beispielsweise eine Durchlassrichtung 14 aufweisen. Alternativ kann ein Sperrelement verwendet werden, das zwei Durchlassrichtungen aufweist.

Das Substrat umfasst weiterhin ein zweites passives Sperrelement 15, das zum Sperren eines Flüssigkeitsaustauschs zwischen der Probenkammer 3 und dem zweiten Flüssigkeitsreservoir 5 ausgebildet und angeordnet ist. Insbesondere ist das zweite passive Sperrelement zwischen dem Fluidkanal 5b und dem Fluidkanal 7 angeordnet und grenzt unmittelbar an den Fluidkanal 5b und den Fluidkanal 7 an. Das Sperrelement 15 ist in dem Bereich 10 angeordnet, in dem der Fluidkanal 5b und der Fluidkanal 7 einander überlappen.

Auch das zweite Sperrelement 15 kann in Form eines Rückschlagventils, beispielsweise in Form eines Schirmventils, ausgebildet sein. Das Sperrelement kann beispielsweise eine Durchlassrichtung 16 aufweisen. Alternativ kann ein Sperrelement verwendet werden, das zwei Durchlassrichtungen aufweist.

In den Figuren 1a und 1b ist außerdem ein erster Anschluss 17, hier in Form einer Öffnung, gezeigt. Über diesen Anschluss kann das Substrat, insbesondere das erste Flüssigkeitsreservoir 4 an ein Pumpensystem angeschlossen werden. Dieses Pumpensystem ist nicht Teil des Substrats. Der Anschluss ist an der oberen Außenwand der Kammer 4a angeordnet, hier in Form einer Öffnung in der oberen Außenwand ausgebildet. Alternativ kann der Anschluss auch an einer Seitenwand oder dem Boden der Kammer vorgesehen sein. Hier ist die obere Außenwand Teil eines abnehmbaren Deckels 8 der Kammer 4a.

Außerdem ist in den Figuren 1a und 1b ein zweiter Anschluss 18, hier in Form einer Öffnung, gezeigt. Über diesen Anschluss kann das Substrat, insbesondere das zweite Flüssigkeitsreservoir 5 an ein Pumpensystem angeschlossen werden. Der Anschluss ist an der oberen Außenwand der Kammer 5a angeordnet, hier in Form einer Öffnung in der oberen Außenwand ausgebildet. Alternativ kann der Anschluss auch an einer Seitenwand oder dem Boden der Kammer vorgesehen sein. Hier ist die obere Außenwand Teil eines abnehmbaren Deckels 8 der Kammer 5a.

Zur Veranschaulichung sind in den Figuren 1a und 1b die Längsachse 19a, die Querachse 19b und die Hochachse 19c des Substrats angedeutet.

In der in den Figuren 1a und 1b gezeigten Ausführungsform ist das Substrat 1 mehrteilig ausgebildet ist und umfasst eine strukturierte Bodenplatte 20 und eine strukturierte Deckplatte 21.

Zudem sind in den Figuren 1a und 1b zwei Öffnungen 22 und 23 gezeigt, die unmittelbar oberhalb der Probenkammer angeordnet sind. In den Figuren sind die Öffnungen in einem mit einem Deckel 22a bzw. 23a verschlossenen Zustand dargestellt. Vor dem Verschließen kann eine der Öffnungen 22 zum Befüllen der Probenkammer mit der Probe verwendet werden. Dabei kann auch die andere Öffnung 23 nicht verschlossen sein und dazu dienen, dass beim oder nach dem Befüllen der Probenkammer Luft aus der Probenkammer entweichen kann. Einer oder beide Deckel 22a und 23a können Anschlüsse für ein Druckluftsystem aufweisen.

Zur Veranschaulichung ist in den Figuren 1a und 1b eine Probe 24, gezeigt, die im bestimmungsgemäßen Gebrauch in der Probenkammer angeordnet sein kann. In den Figuren 1a und 1b ist die Probe beispielhaft in Form einer Vielzahl von Zellen 24a dargestellt.

Auch ein Beobachtungsbereich 25 zum Beobachten der Proben im bestimmungsgemäßen Gebrauch ist in Figur 1b angedeutet. In Figuren 1a und 1b ist auch eine im bestimmungsgemäßen Gebrauch vorgesehene Transportrichtung 29 der Flüssigkeit durch das Fluidsystem mit Pfeilen angedeutet.

In Figur 1a sind zum besseren Verständnis an die Anschlüsse angeschlossene Schläuche 48 und 50 und eine Pumpe 47 gezeigt, die jedoch nicht Teil des Substrats sind.

Optional kann das ganze Substrat oder nur ein Teil des Substrats transparent, insbesondere aus einem transparenten Kunststoffmaterial ausgebildet, sein. Insbesondere kann der Boden der Probenkammer transparent sein. Das Substrat kann insbesondere derart ausgebildet sein, dass die darin befindlichen Proben mikroskopisch untersucht werden können.

Wenn in einem wie in Figuren 1a und 1b gezeigtem Substrat Überdruck an das Flüssigkeitsreservoir 4 angelegt wird, wird Flüssigkeit durch den Fluidkanal 4b und den Fluidkanal 6 in die Kammer und aus der Kammer durch den Fluidkanal 7 in das Flüssigkeitsreservoir 5 transportiert. Die Sperrelemente 13 und 15 sind so angeordnet, dass dies der Öffnungsrichtung entspricht und durch das Anlegen des Überdrucks die Sperrelemente die Durchlassstellung einnehmen. Wird hingegen ein Unterdruck an die Kammer 4 angelegt, so nimmt das Sperrelement 13 eine Sperrstellung ein und es kann keine Flüssigkeit zwischen der Probenkammer und dem Flüssigkeitsreservoir 4 ausgetauscht werden. In der in Figuren 1a und 1b gezeigten Anordnung kann beispielsweise unverbrauchte Prozessflüssigkeit in dem Flüssigkeitsreservoir 4 gelagert werden und bei Bedarf durch die Probenkammer geleitet und anschließend dem Flüssigkeitsreservoir 5 zugeführt werden. Das Flüssigkeitsreservoir 5 kann als Abfallreservoir dienen. Wenn das Substrat mehrere Probenkammern aufweist und/oder Flüssigkeit aus mehreren weiteren Flüssigkeitsreservoiren der Probenkammer zugeführt wird, kann das Flüssigkeitsreservoir 5 auch für Flüssigkeiten aus mehreren der Probenkammern und/oder aus mehreren Flüssigkeitsreservoiren ausgebildet sein. Alternativ oder zusätzlich kann das Substrat auch mindestens ein weiteres Abfallreservoir umfassen.

Figuren 2a bis 2c zeigen beispielhaft die Funktionsweise eines Sperrelements in Form eines Rückschlagventils, hier am Beispiel eines Schirmventils. Zur Veranschaulichung ist in Figuren 2a bis 2c das Sperrelement wie das Sperrelement 13 in den Figuren 1a und 1b angeordnet.

In den Figuren 2a bis 2c ist gezeigt, dass zwischen dem Fluidkanal 4b und dem Fluidkanal 6 im Bereich des Ventils eine horizontal angeordnete Wand 26 angeordnet ist. Die Wand weist eine oder mehrere Verbindungsbohrungen 27 auf, die um eine zentrale Bohrung 28 herum unterhalb des Schirms 13a des Schirmventils angeordnet sind. Das Schirmventil ist mittels der zentralen Bohrung befestigt, insbesondere mittels eines Verankerungselements 13b des Schirmventils, das in der zentralen Bohrung verankert ist.

Abhängig von dem Druck P1, der an dem Fluidkanal 4a anliegt und dem Druck P2, der an dem Fluidkanal 6 anliegt, ändert sich das fluidische Verhalten des Rückschlagventils. Insbesondere ändert sich das fluidische Verhalten des Rückschlagventils abhängig von der Druckdifferenz zwischen P1 und P2. Wenn P1 und P2 gleich sind nimmt das Ventil eine Sperrstellung ein, es ist also geschlossen. Wenn der Druck P2 größer als P1 ist, die Druckdifferenz also einen negativen Wert hat, was als Gegendruck bezeichnet wird, wird das Ventil dadurch zuverlässig in der Sperrstellung gehalten und so die Verschlusswirkung des Ventils unterstützt. Beispielsweise kann das erreicht werden, indem P2 dem Umgebungsdruck entspricht und P1 ein Unterdruck ist, der beispielsweise von einer Pumpe erzeugt wird. Alternativ kann das beispielsweise erreicht werden, wenn P1 dem Umgebungsdruck entspricht und P2 ein Überdruck ist, der beispielsweise von einer Pumpe erzeugt wird. Wenn die Druckdifferenz unterhalb eines Schwellwerts, auch als Öffnungsdruck bezeichnet, liegt, nimmt das Ventil ebenfalls eine Sperrstellung ein, es ist also geschlossen. Das Ventil in Sperrstellung ist in Figur 2a gezeigt. Wenn die Druckdifferenz größer oder gleich dem Schwellwert ist, nimmt das Ventil eine Durchlassstellung ein, es ist also geöffnet. Das Ventil in Durchlassstellung ist in Figur 2b gezeigt.

Im Falle des Schirmventils liegt der Schirm 13a des Schirmventils in der Sperrstellung derart auf der Wand 26 auf, dass keine Flüssigkeit zwischen dem Fluidkanal 4a und dem Fluidkanal 6 ausgetauscht wird. Insbesondere umgibt die Auflagefläche des Schirms alle Verbindungsbohrungen. In der Durchlassstellung ist der Schirm von der Auflagefläche gehoben, so dass Flüssigkeit zwischen dem Fluidkanal 4a und dem Fluidkanal 6 strömen kann. Aufgrund der Druckdifferenz, die zum Öffnen des Schirmventils erforderlich ist, strömt hier die Flüssigkeit in erster Linie von dem Fluidkanal 4a in den Fluidkanal 6.

Figur 2c zeigt eine Draufsicht, in der insbesondere erkennbar ist, wie die Verbindungsbohrungen relativ zu dem Ventil angeordnet sind.

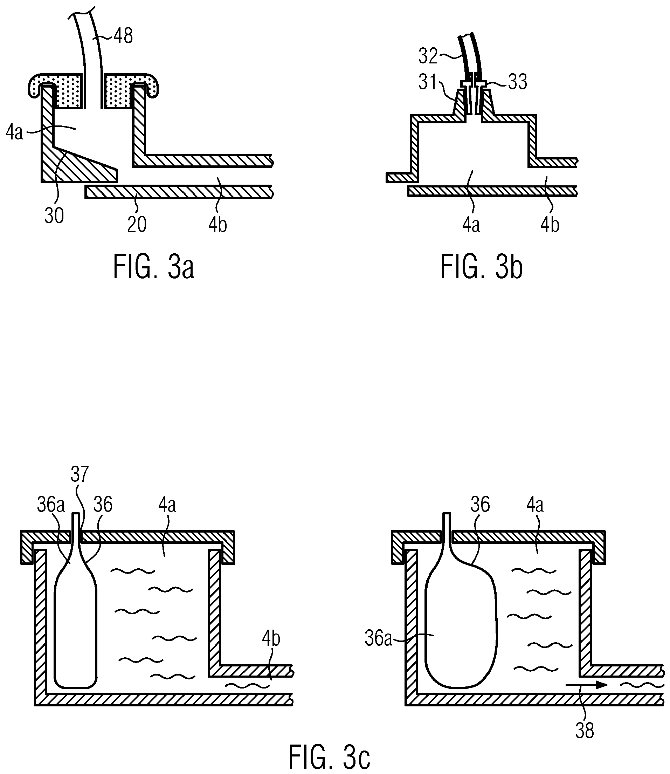

In den Figuren 3a bis 3c sind jeweils Schnittansichten des Substrats im Bereich eines Flüssigkeitsreservoirs gezeigt. Ein erfindungsgemäßes Substrat kann im Bereich einer, mehrerer oder aller Flüssigkeitsreservoire wie in diesen Figuren gezeigt ausgebildet sein.

In Figur 3a ist eine Schnittansicht eines Substrats 1 im Bereich eines Flüssigkeitsreservoirs 4a gezeigt, das im Wesentlichen wie in Figuren 1a und 1b gezeigt ausgebildet ist. Anders als in Figuren 1a und 1b kann der Boden der Kammer derart ausgebildet sein, dass er einen oder mehrere Teilbereiche 30 aufweist, die zu dem Fluidkanal 4b hin geneigt sind. Mit anderen Worten kann der Boden der Kammer Schrägen aufweisen, die zu dem Fluidkanal hin abfallend ausgebildet sind. Insbesondere kann das Fluidreservoir trichterförmig ausgebildet sein. Wenn das Substrat eine Bodenplatte eine Deckplatte umfasst, können der oder die geneigten Teilbereiche insbesondere in der Deckplatte ausgebildet sein, wie in Figur 3a gezeigt.

In Figur 3b ist eine Schnittansicht eines Substrats 1 im Bereich eines Flüssigkeitsreservoirs 4a gezeigt, das im Wesentlichen wie in Figuren 1a und 1b gezeigt ausgebildet ist. Anders als in Figuren 1a und 1b weist das Substrat einen Adapter 31 auf, hier beispielhaften einen weiblichen Luer-Adapter. So kann beispielsweise ein hier nicht gezeigtes Pumpensystem mittels eines Schlauchs 32 an den Adapter angeschlossen werden. Insbesondere kann am Ende des Schlauchs ein männlicher Luer-Adapter 33 angebracht sein, der mit dem weiblichen Luer-Adapter verbunden werden kann. Der Schlauch und der männliche Luer-Adapter sind nicht Teil des Substrats und hier nur zu Veranschaulichung dargestellt. Sie können aber Teil eines erfindungsgemäßen Systems sein. Anders als in Figur 3a, wo der Anschluss in einem angeordnet ist, ist hier der Anschluss in Figur 3b in der Deckplatte des Substrats angeordnet, die die obere Außenwand der Kammer 4a bildet.

In Figur 3c sind Schnittansichten eines Substrats 1 im Bereich der Kammer 4a eines Flüssigkeitsreservoirs gezeigt. Hier ist die Kammer 4a in dem Substrat ausgebildet und in der Kammer ist ein Verdrängungselement 36 in Form eines Beutels angeordnet, der über einen Anschluss 37 des Substrats an ein Pumpensystem angeschlossen werden kann oder eine Kammer eines Flüssigkeitsreservoirs des Fluidkanalsystems bilden kann. Im bestimmungsgemäßen Gebrauch kann durch Vergrößern des Volumens des Beutels 36 Flüssigkeit, die sich in der Kammer 4a befindet, in Richtung des Pfeils 38 aus der Kammer verdrängt werden. Optional kann in dem Beutel befindliche Flüssigkeit oder in dem Beutel befindliches Gas, von Flüssigkeit, die in die Kammer 4a gepumpt wird, aus dem Beutel verdrängt werden.

In der Figur sind verschiedene Zustände des Systems gezeigt, nämlich ein erster Zustand, in dem der Beutel 36 kaum Volumen in der Kammer 4a verdrängt, und ein zweiter Zustand, in dem der Beutel 36 teilweise aufgepumpt ist und in der Kammer 4a so viel Volumen verdrängt, dass Flüssigkeit aus der Kammer 4a in Richtung des Pfeils 38 in den Fluidkanal 4b transportiert wird. Der Beutel kann derart ausgebildet sein, dass er, wenn keine Druckbeaufschlagung vorliegt, schlaff ist. Der Beutel kann derart ausgebildet sein, dass sein Volumen durch Druckbeaufschlagung derart vergrößert werden kann, dass er im Wesentlichen die gesamte Kammer ausfüllt. Der Beutel kann Wände aufweisen, die elastisch sind, insbesondere derart, dass eine durch Füllen mit Flüssigkeit und/oder durch Aufpumpen bewirkte Dehnung der Wände reversibel ist. Der Beutel kann in Form eines Ballons ausgebildet sein.

Die Kammer 4a ist nicht mit dem Pumpensystem verbunden. Das ermöglicht, die Sterilität leichter zu erhalten. Außerdem ist ein von der Lage relativ zum Schwerkraftvektor unabhängiger Transport von Flüssigkeiten einfacher.

Als eine Alternative dazu, dass der Beutel über einen Anschluss des Substrats an eine Pumpe angeschlossen werden kann, kann der Beutel eine der Kammern eines Flüssigkeitsreservoirs des Fluidkanalsystems bilden und mit einem Fluidkanal verbunden sein. Beispielsweise kann so der Transport einer Flüssigkeit in den Beutel den Transport einer Flüssigkeit aus der Kammer 4a heraus bewirken. Insbesondere kann der Beutel als Auffangreservoir für verbrauchte Flüssigkeit dienen.

Als in den Figuren nicht dargestellte Abwandlung der in Figur 3c gezeigten Anordnung kann in einer Aufnahme im Substrat das oben beschriebene Verdrängungselement 36 angeordnet sein und die Kammer 4a des Flüssigkeitsreservoirs kann als zweiter Beutel ausgebildet sein. Der zweite Beutel kann derart ausgebildet sein, dass er, wenn sich darin keine Flüssigkeit befindet und keine Druckbeaufschlagung vorliegt, schlaff ist. Der zweite Beutel kann Wände aufweisen, die elastisch sind, insbesondere derart, dass eine durch Füllen mit Flüssigkeit bewirkte Dehnung der Wände reversibel ist. Der Beutel kann in Form eines Ballons ausgebildet sein.

In der in Figur 3c gezeigten Ausführungsform und ihren Abwandlungen kann für den Beutel 36 ein elastisches Material verwendet werden, das beim Aufpumpen gedehnt wird. Der Beutel erzeugt dann einen Druck, der ein eventuell komprimierbares Medium wie z.B. Luft komprimiert. Für den Fall, dass man anhand der in den Beutel 36 gepumpten Medium-Menge das verdrängte Volumen in der Kammer des Flüssigkeitsreservoirs kontrollieren will, ist es zielführend, den Druck im Beutel 36 kontinuierlich zu messen und entsprechend die zu transportierende Gasmasse auf den jeweiligen Druckwert zu kompensieren. Als Berechnungsgrundlage dient in diesem Fall die ideale Gasgleichung p V = n R T.

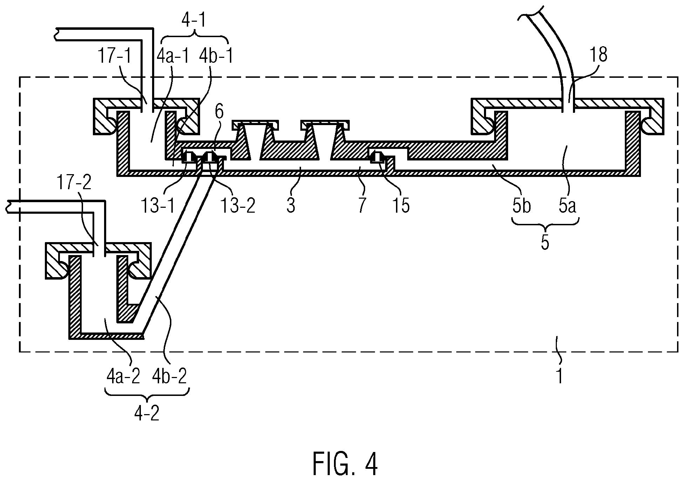

In Figur 4 ist eine zweite Ausführungsform des Substrats gezeigt. Das Substrat kann beispielsweise im Prinzip wie oben beschrieben ausgebildet sein, jedoch statt des Flüssigkeitsreservoirs 4 die Flüssigkeitsreservoire 4-1 und 4-2 umfassen. Das Flüssigkeitsreservoir 4-2 kann eine Kammer 4a-2 und einen Fluidkanal 4b-2 umfassen und über ein Sperrelement 13-2 mit dem Fluidkanal 6 verbunden sein. Das Flüssigkeitsreservoir kann über einen Anschluss 17-2 mit einem Pumpensystem verbunden werden. Alternativ kann es nur mit dem Fluidkanal verbunden sein und durch Verdrängung der Flüssigkeit aus der Kammer ein Flüssigkeitstransport erfolgen, beispielsweise wie in Figur 3c gezeigt. Die Kammern 4a-1 und 4a-2 können, wie hier gezeigt, auf unterschiedlichen Höhen angeordnet sein. Insbesondere kann eine Kammer unterhalb der anderen Kammer angeordnet sein und diese in Draufsicht ganz oder teilweise überlappen. Alternativ können die Kammern auf einer Höhe angeordnet sein. Die Kammern können also nebeneinander angeordnet sein. Die Flüssigkeitsreservoire 4-1 und 4-2, Sperrelemente 13-1 und 13-2 und die Anschlüsse 17-1 und 17-2 können jeweils beispielsweise wie die oben im Zusammenhang mit dem Flüssigkeitsreservoir 4, dem Sperrelement 13 und dem Anschluss 17 beschrieben ausgebildet sein.

Die Funktionsweise des Substrats ist ähnlich zu der oben im Zusammenhang mit Figuren 1a und 1b beschriebenen. An die Flüssigkeitsreservoire 4-1 und 4-2 kann, beispielsweise nacheinander, ein Überdruck angelegt werden, beispielsweise zuerst an das Flüssigkeitsreservoir 4-1 und dann an das Flüssigkeitsreservoir 4-2, während jeweils an dem anderen Flüssigkeitsreservoir kein Überdruck oder ein Unterdruck anliegt. Dadurch wird Flüssigkeit erst aus dem ein Flüssigkeitsreservoir 4-1 in die Probenkammer 7 transportiert. Wenn sich bereits Flüssigkeit, beispielsweise eine erste Prozessflüssigkeit, in der Probenkammer befindet, wird diese durch die Flüssigkeit aus dem Flüssigkeitsreservoir 4-1 verdrängt und in das Flüssigkeitsreservoir 5 transportiert. Wird dann Überdruck an das Flüssigkeitsreservoir 4-2 angelegt, wird die Flüssigkeit aus diesem Flüssigkeitsreservoir in die Probenkammer transportiert. Sofern dort noch Flüssigkeit vorhanden ist, wird diese ebenfalls verdrängt und beispielsweise in das Flüssigkeitsreservoir 5 transportiert. Auf diese Weise kann beispielsweise ein Austausch von Prozessflüssigkeiten in der Probenkammer erfolgen. Vorzugsweise ist dann die Flüssigkeit im Flüssigkeitsreservoir 4-1 eine Puffer- oder Spülflüssigkeit und die Flüssigkeit im Flüssigkeitsreservoir 4-2 eine zweite Prozessflüssigkeit.

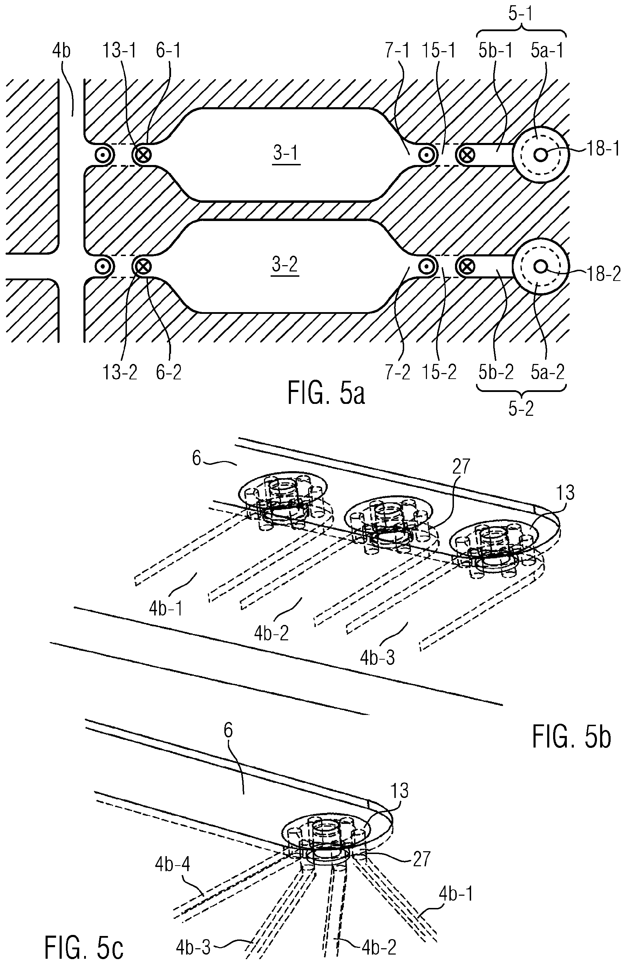

In Figur 5a ist ein Teilbereich eines Substrats gezeigt, bei dem an den Fluidkanal 4b des ersten Flüssigkeitsreservoirs 4 mehrere, hier beispielhaft zwei, Fluidkanäle 6-1 und 6-2 angrenzen. Nur zur Veranschaulichung ist hier eine Darstellung gewählt, bei der die Fluidkanäle 6-1 und 6-2 nichtüberlappend mit dem Fluidkanal 4b, sondern nach rechts versetzt, dargestellt sind, so dass die Durchlassrichtungen und Sperrrichtungen der Sperrelemente 13-1 und 13-2 besser erkennbar sind. Zwischen dem Fluidkanal 4b und den Fluidkanälen 6-1 und 6-2 ist jeweils ein Sperrelement 13-1 und 13-2 angeordnet, beispielsweise eines der oben beschriebenen Sperrelemente. Das Substrat umfasst mehrere, hier beispielsweise zwei, Probenkammern 3-1 und 3-2, wobei der Fluidkanal 6-1 in die Probenkammer 3-1 mündet und der Fluidkanal 6-2 in die Probenkammer 3-2 mündet.

Das Substrat umfasst weiterhin zwei Flüssigkeitsreservoire 5-1 und 5-2, die jeweils eine Kammer 5a-1, 5a-2 und einen Kanal 5b-1, 5b-2 umfassen. Das Substrat umfasst weiterhin einen Fluidkanal 7-1, der an den Fluidkanal 5b-1 des Flüssigkeitsreservoirs 5-1 angrenzt, wobei dazwischen ein Sperrelement 15-1 angeordnet ist, und einen Fluidkanal 7-2, der an den Fluidkanal 5b-2 des Flüssigkeitsreservoirs 5-2 angrenzt, wobei dazwischen ein Sperrelement 15-2 angeordnet ist.

Das Substrat ist derart ausgebildet, dass Flüssigkeitsreservoir 5-1 und Flüssigkeitsreservoir 5-2 unabhängig voneinander mit Druck beaufschlagt werden können, beispielsweise jeweils über einen eigenen Anschluss 18-1, 18-2.

Der Fluidkanal 7-1 mündet in die Probenkammer 3-1 und der Fluidkanal 7-2 mündet in die Probenkammer 3-2. Die Flüssigkeitsreservoire und Probenkammern können jeweils wie oben beschrieben ausgebildet und angeordnet sein. Die Probenkammern können auf derselben Höhe angeordnet sein.

Alternativ zu der in der Figur 5a gezeigten Variante, in der mehrere Probenkammern an einen gemeinsamen ersten Fluidkanal 4b und an separate zweite Fluidkanäle 5b-1, 5b-2 angeschlossen sind, können mehrere Probenkammern an einen gemeinsamen zweiten Fluidkanal 5b und an separate erste Fluidkanäle und entsprechende Flüssigkeitsreservoire angeschlossen sein. Das Substrat kann dann derart ausgebildet sein, dass diese Flüssigkeitsreservoire unabhängig voneinander mit Druck beaufschlag werden können.

Beide Alternativen ermöglichen einen selektiven Flüssigkeitstransport, bei reduzierter Anzahl an Kanalstrukturen.

Beispielhaft kann das Substrat wie in der europäischen Patentanmeldung mit dem Aktenzeichen 20160085.5 gezeigte Anordnung von Kammern und Fluidkanälen aufweisen, wobei an einer oder mehreren der Mündungen der dort gezeigten Kanäle oder Kammern ein erfindungsgemäßes Sperrelement angeordnet sein kann. Die Sperrelemente ermöglichen, die Flüssigkeit in den in besagter Patentanmeldung beschriebenen Zellkulturbereichen über länger Zeiträume vor diffusionsbedingter Vermischung mit anderen Flüssigkeiten zu schützen.

In Figur 5b ist ein Teil eines Substrats mit mehreren Flüssigkeitsreservoiren gezeigt, die jeweils einen Fluidkanal 4b-1, 4b-2 und 4b-3 umfassen. Jeder der Fluidkanäle grenzt an den Fluidkanal 6 an, wobei dazwischen jeweils ein Sperrelement 13 angeordnet ist. Im hier dargestellten Beispiel handelt es sich beispielsweise um ein Ventil, wie es in den Figuren 2a bis 2c gezeigt ist, aber andere Sperrelemente können stattdessen verwendet werden. Der Fluidkanal 6 ist, beispielsweise wie in Figur 1a und 1b gezeigt, fluidisch mit der Probenkammer verbunden.

In Figur 5c umfasst das Substrat mehrere Flüssigkeitsreservoire, die jeweils einen Fluidkanal 4b-1 bis 4b-4 umfassen, wobei die Fluidkanäle 4b-1 bis 4b-4 über ein einziges Sperrelement 13 mit dem Fluidkanal 6 verbunden sind. Dies ist eine besonders platzsparende Ausführungsform.

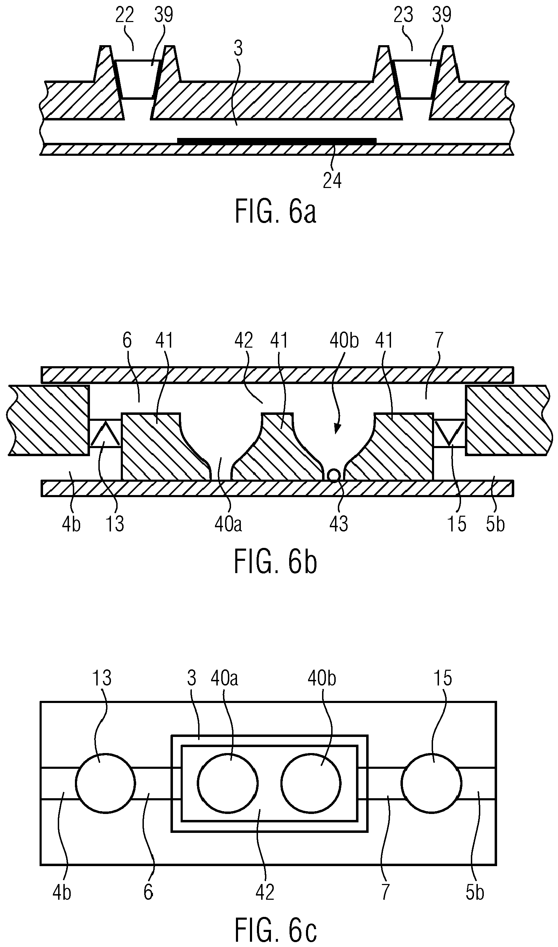

Figur 6a zeigt ein Beispiel für einen Teil eines Substrats, insbesondere der Probenkammer 3. Oberhalb der Probenkammer sind Öffnungen 22 und 23 angeordnet, über die Proben, beispielsweise Zellen in die Probenkammer eingebracht werden können und/oder durch die vor dem Verschließen Luft entweichen kann. Die Öffnungen können beispielsweise jeweils mit einem Stopfen 39 verschlossen werden. Die Öffnungen und Stopfen können beispielsweise konisch ausgebildet sein. Abgesehen davon kann das Substrat beispielsweise wie oben beschrieben ausgebildet sein. Im vorliegenden Beispiel ist gezeigt, dass in einem Teilbereich des Bodens der Probenkammer eine Probe 24, insbesondere in Form von Zellen, angeordnet ist. Insbesondere kann der Boden im bestimmungsgemäßen Gebrauch mit adhärenten Zellen bewachsen sein. Der Teilbereich des Bodens ist zwischen den Öffnungen 22 und 23 angeordnet und kann als Beobachtungsbereich betrachtet werden. Der Boden kann insbesondere in Form eines Deckglases ausgebildet sein, durch das hindurch die Zellen mikroskopiert werden können.

Figur 6b zeigt ein Beispiel für einen Teil eines Substrats, insbesondere der Probenkammer 3, in einer Schnittansicht. Figur 6c zeigt den Teil des Substrats, der in Figur 6b in einer Schnittansicht gezeigt ist in Draufsicht. Die Probenkammer weist mehrere Reservoire 40a und 40b in Form von Töpfchen auf, die sich hier jeweils nach unten hin verjüngen. Die Töpfchen sind seitlich durch Wände 41 begrenzt. Die Probenkammer weist auch einen oben an die Töpfchen angrenzenden Fluidkanal 42 auf. Die Fluidkanäle 6 und 7 münden in den Fluidkanal 42. Flüssigkeit kann so beispielsweise nacheinander durch den Fluidkanal 6, den Fluidkanal 42 und den Fluidkanal 7 transportiert werden. Der Fluidkanal 42 ist derart ausgebildet und angeordnet, dass beim Transport von Flüssigkeit durch den Fluidkanal 42 die Flüssigkeit oben an den Töpfchen entlang strömt. Wenn im bestimmungsgemäßen Gebrauch Zellen in den Töpfchen angeordnet sind, werden die Zellen dadurch superfundiert. Abgesehen davon kann das Substrat beispielsweise wie oben beschrieben ausgebildet sein.

Die Probenkammer kann zunächst offen sein. Dann kann die Probe, beispielsweise Zellen, in die Töpfchen eingebracht werden und die Probenkammer verschlossen werden, beispielsweise mit einem Deckglas. Das Deckglas kann beispielsweise mit Doppelklebeband befestigt werden. Die Zellen können optional in Form von Sphäroiden 43 in die Töpfchen eingebracht werden oder in den Töpfchen Sphäroide ausbilden.

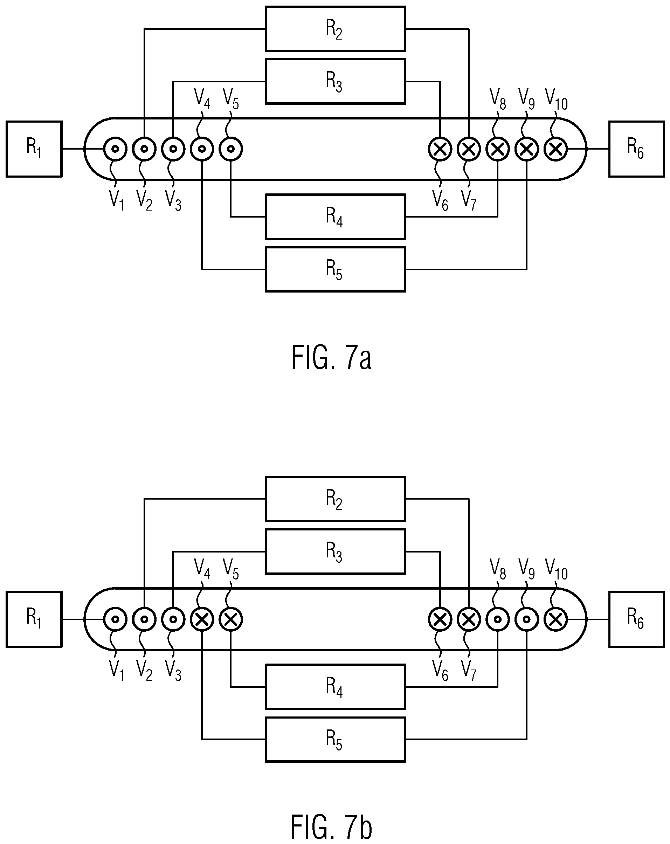

Figuren 7a und 7b zeigen jeweils schematisch in Draufsicht ein Beispiel für ein Substrat mit sechs Flüssigkeitsreservoiren R1, R2, R3, R4, R5 und R6. Jedes der Reservoire kann beispielsweise wie oben im Zusammenhang mit den Flüssigkeitsreservoiren 4 und 5 beschrieben ausgebildet sein. Jedes der Flüssigkeitsreservoire ist zumindest mittels einer fluidischen Verbindung über ein Sperrelement mit der Probenkammer verbunden. Jedes der Flüssigkeitsreservoire R1 und R6 ist jeweils nur über eine fluidische Verbindung mit der Probenkammer verbunden. Das Flüssigkeitsreservoir R1 über das Sperrelement V1 mit der Probenkammer verbunden, das Flüssigkeitsreservoir R6 ist über das Sperrelement V10 mit der Probenkammer verbunden. Das Flüssigkeitsreservoir R2 weist zwei fluidische Verbindungen zur Probenkammer auf, eine über das Sperrelement V2 und eine über das Sperrelement V7, wobei die Sperrelemente V2 und V7 Rückschlagventile sein können, wobei eines der Rückschlagventile eine Durchlassrichtung zur Probenkammer hin und das andere der Rückschlagventile eine Durchlassrichtung aus der Probenkammer heraus haben kann. Das Flüssigkeitsreservoir R3 weist zwei fluidische Verbindungen zur Probenkammer auf, eine über das Sperrelement V3 und eine über das Sperrelement V6, wobei die Sperrelemente V3 und V6 Rückschlagventile sein können, wobei eines der Rückschlagventile eine Durchlassrichtung zur Probenkammer hin und das andere der Rückschlagventile eine Durchlassrichtung aus der Probenkammer heraus haben kann. Das Flüssigkeitsreservoir R4 weist zwei fluidische Verbindungen zur Probenkammer auf, eine über das Sperrelement V5 und eine über das Sperrelement V8, wobei die Sperrelemente V5 und V8 Rückschlagventile sein können, wobei eines der Rückschlagventile eine Durchlassrichtung zur Probenkammer hin und das andere der Rückschlagventile eine Durchlassrichtung aus der Probenkammer heraus haben kann. Das Flüssigkeitsreservoir R5 weist zwei fluidische Verbindungen zur Probenkammer auf, eine über das Sperrelement V4 und eine über das Sperrelement V9, wobei die Sperrelemente V4 und V9 Rückschlagventile sein können, wobei eines der Rückschlagventile eine Durchlassrichtung zur Probenkammer hin und das andere der Rückschlagventile eine Durchlassrichtung aus der Probenkammer heraus haben kann.

Die Flüssigkeitsreservoire können derart ausgebildet und angeordnet sein, dass die Fluidkanäle einander nicht kreuzen. Alternativ oder zusätzlich können die Flüssigkeitsreservoire derart ausgebildet und angeordnet sein, dass die Fluidkanäle alle die gleiche Länge aufweisen. Alternativ oder zusätzlich können die Flüssigkeitsreservoire derart ausgebildet und angeordnet sein, dass die Fluidkanäle alle denselben fluidischen Widerstand aufweisen.

Die Figuren 7a und 7b unterscheiden sich jeweils in der Durchlassrichtung der Ventile, wobei die eine Richtung durch Punkte und die andere durch Kreuze gekennzeichnet ist. Durch die Anordnung der Ventile ergeben sich die Strömungsrichtungen im bestimmungsgemäßen Gebrauch.

Im Folgenden werden diese Strömungsrichtungen beispielhaft beschrieben. Wenn an dem Flüssigkeitsreservoir R1 Überdruck und an dem Reservoir R6 zugleich Unterdruck angelegt wird, strömt Flüssigkeit durch das Ventil V1 in die Probenkammer und durch das Ventil V10 in das Flüssigkeitsreservoir R6.

Wenn an dem Flüssigkeitsreservoir R2 Überdruck und an dem Reservoir R3 zugleich Unterdruck angelegt wird, strömt Flüssigkeit durch das Ventil V2 in die Probenkammer und durch das Ventil V6 in das Flüssigkeitsreservoir R3.

Wenn an dem Flüssigkeitsreservoir R3 Überdruck und an dem Reservoir R2 zugleich Unterdruck angelegt wird, strömt Flüssigkeit durch das Ventil V3 in die Probenkammer und durch das Ventil V7 in das Flüssigkeitsreservoir R2. Das gilt analog für die Flüssigkeitsreservoire R4 und R5.

Wenn die Flüssigkeitsreservoire und die Ventile wie in Figur 7a ausgebildet und angeordnet sind, strömt dabei die Flüssigkeit beim Transport von R2 nach R3 in dieselbe Richtung durch die Probenkammer wie beim Transport von R3 nach R2. Das gilt analog für die Flüssigkeitsreservoire R4 und R5. Außerdem strömt die Flüssigkeit beim Transport von R2 nach R3 in dieselbe Richtung durch die Probenkammer wie beim Transport von R4 nach R5. Außerdem strömt die Flüssigkeit beim Transport von R2 nach R3 in dieselbe Richtung durch die Probenkammer wie beim Transport von R1 nach R6.

Wenn die Flüssigkeitsreservoire und die Ventile wie in Figur 7b ausgebildet und angeordnet sind, strömt die Flüssigkeit beim Transport von R2 nach R3 in dieselbe Richtung durch die Probenkammer wie beim Transport von R3 nach R2. Das gilt analog auch für die Flüssigkeitsreservoire R4 und R5. Anders als bei der Anordnung in Figur 7a strömt die Flüssigkeit aber beim Transport von R2 nach R3 in die entgegengesetzte Richtung durch die Probenkammer als beim Transport von R4 nach R5.

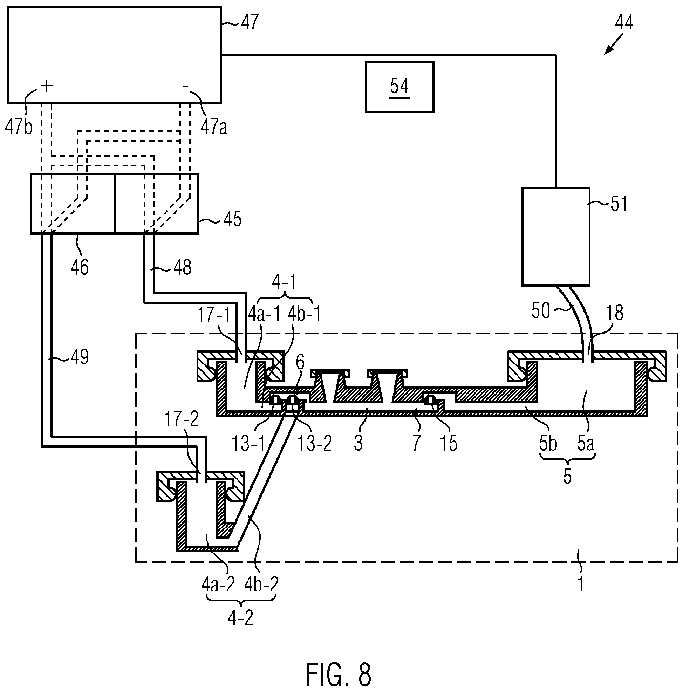

In Figur 8 ist beispielhaft ein erfindungsgemäßes System 44 mit einem Substrat 1 gezeigt, wie es im Zusammenhang mit Figur 4 beschrieben wurde. Alternativ kann das System ein anderes der oben beschriebenen Substrate, beispielsweise wie im Zusammenhang mit Figur 1 beschrieben, oder ein anderes erfindungsgemäßes Substrat umfassen.

Das System umfasst in dieser Ausführungsform Ventile 45 und 46 und eine Pumpe 47 mit einem Unterdruckausgang 47a und einem Überdruckausgang 47b, die im bestimmungsgemäßen Gebrauch jeweils mit einem oder beiden der Ventile 45 und 46 verbunden sind. Das System umfasst weiterhin Schläuche 48 und 49, die im bestimmungsgemäßen Gebrauch jeweils mit einem der Ventile 45 und 46 und mit einem der Anschlüsse 17-1 und 17-2 des Substrats verbunden sind. Ein Schlauch 50 ist mit dem Anschluss 18 des Substrats verbunden. Auch dieser Schlauch kann optional über ein Ventil 51 mit der Pumpe 47 verbunden sein.

Das System kann eine Steuervorrichtung 54 aufweisen, die zum Ansteuern eines oder mehrerer der Ventile und/oder einer oder mehrerer der Pumpen ausgebildet ist.

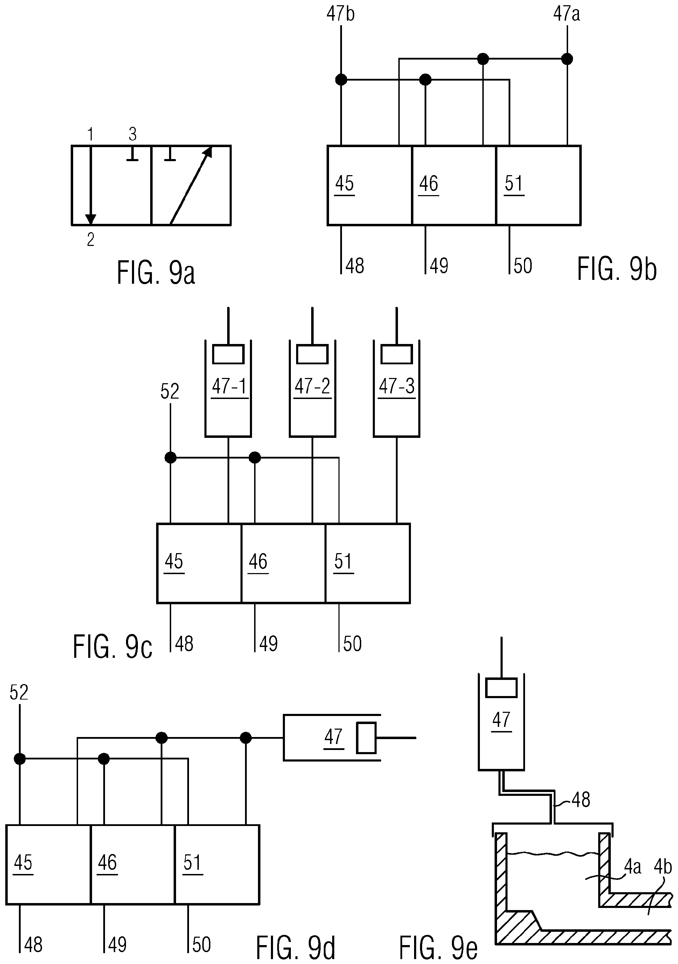

In Figur 9a ist eine schematische Darstellung eines Ventils gezeigt, das als Ventil 45, Ventil 46 und/oder Ventil 51 verwendet werden kann. Es handelt sich dabei um ein 3/2-Wege-Ventil. Ein erster Anschluss des Ventils kann beispielsweise mit dem Überdruckausgang 47b der Pumpe verbunden werden und ein zweiter Anschluss kann mit dem Substrat verbunden werden. Ein dritter Anschluss des Ventils kann optional mit dem Unterdruckausgang 47a der Pumpe verbunden werden. Alternativ kann der dritte Anschluss mit der Umgebungsluft oder mit einer weiteren Pumpe verbunden sein. Über den dritten Anschluss kann, wenn er an eine Pumpe angeschlossen ist, im Betrieb ein Unterdruck an das jeweilige Flüssigkeitsreservoir angelegt werden. Dadurch kann an dem Sperrelement die Verschlusswirkung gefördert werden.

In Figur 9b ist gezeigt, wie die Ventilschaltung aussehen kann, wenn jedes der Ventile 45, 46 und 51 über zwei seiner Anschlüsse mit der Pumpe 47, genauer mit dem Unterdruckausgang 47a und dem Überdruckausgang 47b, verbunden ist. In Figur 9c ist gezeigt, wie die Ventilschaltung aussehen kann, wenn jedes der Ventile mit einem seiner Anschlüsse an einer Pumpe 52, beispielsweise zum Erzeugen von Unterdruck, und mit einem anderen seiner Anschlüsse jeweils mit einer weiteren Pumpe 47-1, 47-2 und 47-3, beispielsweise zum Erzeugen eines Überdrucks zum Transport verbunden ist. das hat den Vorteil, dass der Überdruck, der zum Antreiben der Flüssigkeit im Substrat verwendet wird, für jedes der Flüssigkeitsreservoire unabhängig bereitgestellt werden kann, der Unterdruck, der das Verschließen des Sperrelements unterstützt jedoch einfach durch eine gemeinsame Pumpe bereitgestellt werden kann.

In Figur 9d ist gezeigt, wie die Ventilschaltung aussehen kann, wenn jedes der Ventile 45, 46 und 51 mit einem seiner Anschlüsse mit der Pumpe 47 und mit einem anderen seiner Anschlüsse mit der weiteren Pumpe 52 verbunden ist, wobei die die Pumpe 47 zum Bereitstellen des Überdrucks und die Pumpe 52 zum Bereitstellen des Unterdrucks vorgesehen sein können.

Alternativ oder zusätzlich kann, wie in Figur 9e vereinfacht dargestellt, zumindest eines der Flüssigkeitsreservoire 4 über einen Schlauch 48 direkt mit einer Pumpe 47 verbunden sein, also ohne ein zwischengeschaltetes Ventil.

Die Pumpen 47, 47-1, 47-2, 47-3 und/oder 52 können jeweils in Form einer Kolbenpumpe, Peristaltikpumpe, Membranpumpe oder Schraubenpumpe ausgebildet sein. Insbesondere für die Pumpe, die zum Anlegen des Überdrucks vorgesehen ist, ist es vorteilhaft, wenn die Pumpe derart ausgebildet ist, dass damit kontrollierbare Volumina transportiert werden können.

Alle an das Substrat angeschlossenen Pumpen und gegebenenfalls Ventile und Schläuche bilden zusammen das Pumpensystem 53.

Hinsichtlich der Funktion des Systems ist zu beachten, dass abhängig von den verwendeten Sperrelementen bestimmte Ausgestaltungen und Betriebsweisen vorteilhafter sein können als andere. Beispielsweise ist bei einem Entenschnabelventil vorteilhaft, zum sicheren Schließen des Ventils einen Gegendruck anzulegen.

Ein erfindungsgemäßes Verfahren umfasst, dass an das Fluidsystem 2 eines der oben beschriebenen Substrate 1, beispielsweise mittels eines der oben beschriebenen Systeme 44, genauer mittels eines der oben beschriebenen Pumpensystem 53 Druck angelegt wird. Insbesondere kann der Druck so angelegt werden, dass das Sperrelement 13 oder eines der Sperrelemente 13-1 und 13-2 eine Durchlassstellung einnimmt, also geöffnet ist, und Flüssigkeit aus dem Flüssigkeitsreservoir 4 oder einem der Flüssigkeitsreservoire 4-1 und 4-2 in die Probenkammer 3 transportiert wird. Der Druck kann optional so angelegt werden, dass, insbesondere gleichzeitig, auch das Sperrelement 15 eine Durchlassstellung einnimmt und Flüssigkeit aus der Probenkammer 3 in das Flüssigkeitsreservoir 5 transportiert wird. So kann beispielsweise eine bereits in der Probenkammer befindliche Flüssigkeit durch die Flüssigkeit aus dem Flüssigkeitsreservoir 4, 4-1 oder 4-2 verdrängt werden und in das Flüssigkeitsreservoir 5 transportiert werden.

Optional kann, wenn das Substrat beispielsweise mindestens drei Flüssigkeitsreservoire 4-1, 4-2 und 5 umfasst, zunächst Flüssigkeit aus einem der Flüssigkeitsreservoire 4-1 wie oben beschrieben in die Probenkammer transportiert werden, wobei insbesondere durch Verdrängung eine bereits in der Probenkammer befindliche Flüssigkeit in das Flüssigkeitsreservoir 5 transportiert wird, und anschließend kann Flüssigkeit aus dem anderen der Flüssigkeitsreservoire 4-2 in die Probenkammer transportiert werden und die in der Probenkammer befindliche Flüssigkeit verdrängen, so dass diese beispielsweise in das Flüssigkeitsreservoir 5 oder ein anderes Flüssigkeitsreservoir, insbesondere in das Flüssigkeitsreservoir 4-1, transportiert wird. So erfolgt ein Flüssigkeitsaustausch in der Probenkammer. Insbesondere kann die initial in der Probenkammer befindliche Flüssigkeit eine erste Prozessflüssigkeit sein, die aus dem Flüssigkeitsreservoir 4-1 zugeführte Flüssigkeit eine Spülflüssigkeit oder Pufferflüssigkeit und die aus dem Flüssigkeitsreservoir 4-2 zugeführte Flüssigkeit eine zweite Prozessflüssigkeit. Dadurch kann sichergestellt werden, dass sich nicht erste und zweite Prozessflüssigkeit zugleich in der Probenkammer befinden. Der Austausch der Prozessflüssigkeiten erfolgt somit in einer zuverlässigeren Weise.

Während die Flüssigkeit oder Flüssigkeiten sich in der Probenkammer befinden oder durch die Probenkammer transportiert werden, kann sich in der Probenkammer eine Probe befinden und optional eine Untersuchung der Probe, beispielsweise eine mikroskopische Untersuchung durch den Boden der Probenkammer, durchgeführt werden.

Das Verfahren kann vor den oben beschriebenen Schritten umfassen, dass eine Probe, insbesondere unmittelbar durch die Öffnungen 22 oder 23, in die Probenkammer eingebracht wird und/oder dass die Flüssigkeit bzw. Flüssigkeiten in die Flüssigkeitsreservoire und/oder in die Probenkammer eingefüllt werden. Das Verfahren kann umfassen, dass nach dem Einbringen der Probe und gegebenenfalls einer Flüssigkeit in die Probenkammer die Probenkammer nach außen verschlossen wird, beispielsweise mittels eines Deckels 22a, 23a oder eines Stopfens 39. Alternativ oder zusätzlich kann das Verfahren umfassen, dass, gegebenenfalls nach dem Einfüllen von Flüssigkeit in das jeweilige Flüssigkeitsreservoir, die Flüssigkeitsreservoire verschlossen werden, so dass sie nur noch über die Anschlüsse nach außen offen sind. Das Verfahren kann auch umfassen, dass an die Anschlüsse das Pumpensystem angeschlossen wird.

Das Verfahren kann umfassen, dass nach dem Verschließen der Probenkammer, insbesondere zumindest bis die vor dem Verschließen in das Flüssigkeitsreservoir 4 oder 4-1 oder 4-2 gefüllte Flüssigkeit, aus dem Flüssigkeitsreservoir 4 oder einem oder beiden Flüssigkeitsreservoiren 4-1 und 4-2 in die Probenkammer transportiert wurde, keine Flüssigkeit mehr in das Substrat eingebracht wird. Insbesondere kann das Verfahren umfassen, dass nach dem Verschließen der Probenkammer keine Flüssigkeit mehr in das Substrat eingebracht wird, bis die Flüssigkeit aus allen Flüssigkeitsreservoiren, in die vor dem Verschließen Flüssigkeit eingefüllt wurde, in die Probenkammer transportiert wurde.

Es versteht sich, dass in den zuvor beschriebenen Ausführungsbeispielen genannte Merkmale nicht auf diese speziellen Kombinationen beschränkt sind und auch in beliebigen anderen Kombinationen möglich sind.

## Patentansprüche

1. Substrat (1) zur Untersuchung von Proben (24), insbesondere Zellen oder Molekülen, wobei das Substrat (1) ein Fluidsystem (2) umfasst, das eine in dem Substrat (1) ausgebildete Probenkammer (3) zur Aufbewahrung und Untersuchung von Proben (24) und mindestens ein mit der Probenkammer (3) fluidisch verbundenes Flüssigkeitsreservoir (4, 5) umfasst, und wobei das Substrat (1) mindestens ein passives Sperrelement (13, 15) umfasst, das eine Sperrstellung und eine Durchlassstellung einnehmen kann, wobei in der Sperrstellung ein Flüssigkeitsaustausch zwischen der Probenkammer (3) und dem Flüssigkeitsreservoir (4, 5) gesperrt ist.

2. Substrat (1) nach Anspruch 1, wobei das passive Sperrelement (13, 15) derart ausgebildet ist, dass es abhängig von den Druckverhältnissen in dem Fluidsystem (2) die Sperrstellung oder die Durchlassstellung einnimmt.

3. Substrat (1) nach Anspruch 1 oder 2, wobei in dem Substrat (1) keine aktiven Komponenten angeordnet sind, insbesondere keine aktiven Ventile und/oder Pumpen.

4. Substrat (1) nach einem der vorangegangenen Ansprüche,
wobei das Flüssigkeitsreservoir (4, 5) derart verschließbar ist, dass es keine direkte Verbindung nach außen hat, insbesondere nach außen komplett verschlossen ist, und/oder wobei die Probenkammer (3) derart verschließbar ist, dass sie keine direkte Verbindung nach außen hat, insbesondere nach außen komplett verschlossen ist,
insbesondere, wobei das gesamte Fluidsystem (2) derart verschließbar ist, dass es keine direkte Verbindung nach außen hat, insbesondere nach außen komplett verschlossen ist.

5. Substrat (1) nach einem der vorangegangenen Ansprüche, wobei das Substrat (1) mindestens zwei passive Sperrelemente (13, 15) umfasst, insbesondere wobei das Substrat (1) derart ausgebildet ist, dass die Sperrelemente (13, 15) unabhängig voneinander die Sperrstellung oder die Durchlassstellung einnehmen.

6. Substrat (1) nach einem der vorangegangenen Ansprüche,
wobei das Fluidsystem (2) mehrere mit der Probenkammer (3) fluidisch verbundene Flüssigkeitsreservoire (4, 5) umfasst und das Substrat (1) mehrere passive Sperrelemente (13, 15) umfasst, wobei in der Sperrstellung der Sperrelemente (13, 15) jeweils ein Flüssigkeitsaustausch zwischen der Probenkammer (3) und einem der Flüssigkeitsreservoire (4, 5) gesperrt ist und/oder ein Flüssigkeitsaustausch zwischen den Flüssigkeitsreservoiren (4, 5) gesperrt ist,
insbesondere wobei das Substrat (1) derart ausgebildet ist, dass die Sperrelemente (13, 15) unabhängig voneinander die Sperrstellung oder die Durchlassstellung einnehmen.

7. Substrat (1) nach einem der vorangegangenen Ansprüche,
wobei das Fluidsystem (2) einen ersten Fluidkanal (4b, 5b), der Teil des Flüssigkeitsreservoirs (4, 5) oder mit diesem unmittelbar fluidisch verbunden ist, und einen zweiten Fluidkanal (6, 7) umfasst, der mit der Probenkammer (3) unmittelbar fluidisch verbunden ist,
wobei der erste Fluidkanal (4b, 5b) unmittelbar an den zweiten Fluidkanal (6, 7) angrenzt und das Sperrelement (13, 15) zwischen dem ersten Fluidkanal (4b, 5b) und dem zweiten Fluidkanal (6, 7) angeordnet ist,
insbesondere, wobei das Fluidsystem (2) mindestens eine erste Probenkammer (3-1) und eine zweite Probenkammer (3-2) und mindestens zwei zweite Fluidkanäle (6-1, 6-2, 7-1, 7-2) umfasst, wobei der erste Fluidkanal (4b, 5b) über einen der zweiten Fluidkanäle (6-1, 7-1) mit der ersten Probenkammer (3-1) und über einen anderen der zweiten Fluidkanäle (6-2, 7-2) mit der zweiten Probenkammer (3-2) verbunden ist.

8. Substrat (1) nach einem der vorangegangenen Ansprüche,
wobei das Fluidsystem (2) mindestens ein erstes Sperrelement (13-1) und ein zweites Sperrelement (13-2), mindestens ein erstes Flüssigkeitsreservoir (4-1) und ein zweites Flüssigkeitsreservoir (4-2), mindestens zwei erste Fluidkanäle (4b-1, 4b-2) und einen zweiten Fluidkanal (6), der mit der Probenkammer (3) unmittelbar fluidisch verbunden ist, umfasst,
wobei einer der ersten Fluidkanäle (4b-1) Teil des ersten Flüssigkeitsreservoirs (4-1) oder mit diesem unmittelbar fluidisch verbunden ist und ein anderer der ersten Fluidkanäle (4b-2) Teil des zweiten Flüssigkeitsreservoirs (4-2) oder mit diesem unmittelbar fluidisch verbunden ist, und
wobei die ersten Fluidkanäle (4b-1, 4b-2) unmittelbar an den zweiten Fluidkanal (6) angrenzen und das erste Sperrelement (13-1) zwischen dem einen der ersten Fluidkanäle (4b-1) und dem zweiten Fluidkanal (6) angeordnet ist und das zweite Sperrelement (13-2) zwischen dem anderen der ersten Fluidkanäle (4b-2) und dem zweiten Fluidkanal (6) angeordnet ist,
insbesondere, wobei das Fluidsystem (2) mindestens eine erste Probenkammer (3-1) und eine zweite Probenkammer (3-2) und mindestens zwei zweite Fluidkanäle (6-1, 6-2) umfasst, wobei mindestens einer der ersten Fluidkanäle (4b-1, 4b-2) über einen der zweiten Fluidkanäle (6-1) mit der ersten Probenkammer (3-1) und über einen anderen der zweiten Fluidkanäle (6-2) mit der zweiten Probenkammer (3-2) verbunden ist.

9. Substrat (1) nach einem der vorangegangenen Ansprüche,
wobei das Fluidsystem (2) mindestens ein erstes Sperrelement (13) und ein zweites Sperrelement (15), mindestens ein erstes Flüssigkeitsreservoir (4) und ein zweites Flüssigkeitsreservoir (5), mindestens zwei erste Fluidkanäle (4b, 5b) und mindestens zwei zweite Fluidkanäle (6, 7), die mit der Probenkammer (3) unmittelbar fluidisch verbunden sind, umfasst,
wobei einer der ersten Fluidkanäle (4b) Teil des ersten Flüssigkeitsreservoirs (4) oder mit diesem unmittelbar fluidisch verbunden ist und unmittelbar an einen der zweiten Fluidkanäle (6) angrenzt und dazwischen das erste Sperrelement (13) angeordnet ist,
wobei ein anderer der ersten Fluidkanäle (5b) Teil des zweiten Flüssigkeitsreservoirs (5) oder mit diesem unmittelbar fluidisch verbunden ist und unmittelbar an einen anderen der zweiten Fluidkanäle (7) angrenzt und dazwischen das zweite Sperrelement (15) angeordnet ist,
insbesondere wobei das erste Sperrelement (13) nur eine Durchlassrichtung von dem ersten Flüssigkeitsreservoir (4) in die Probenkammer (3) und das zweite Sperrelement (15) nur eine Durchlausrichtung aus der Probenkammer (3) in Richtung des zweiten Flüssigkeitsreservoirs (5) hat, und/oder
insbesondere wobei das erste Sperrelement (13) oberhalb des an das erste Sperrelement (13) angrenzenden ersten Fluidkanals (4b) und unterhalb des an das erste Sperrelement (13) angrenzenden zweiten Fluidkanals (6) angeordnet ist und/oder wobei das zweite Sperrelement (15) unterhalb des an das zweite Sperrelement (15) angrenzenden ersten Fluidkanals (5b) und oberhalb des an das zweite Sperrelement (15) angrenzenden zweiten Fluidkanals (7) angeordnet ist.

10. Substrat (1) nach einem der Ansprüche 7 bis 9,
wobei der erste Fluidkanal (4b, 5b) und der zweite Fluidkanal (6, 7), in einem Bereich, in dem der erste Fluidkanal (4b, 5b) unmittelbar an den zweiten Fluidkanal (6, 7) angrenzt, in Draufsicht auf das Substrat (1) einander überlappend angeordnet sind und der erste Fluidkanal (4b, 5b) oberhalb oder unterhalb des zweiten Fluidkanals (6, 7) angeordnet ist,
insbesondere, wobei das Substrat (1) mindestens zwei erste Fluidkanäle (4b, 5b) und zwei zweite Fluidkanäle (6, 7) aufweist, wobei je einer der ersten Fluidkanäle (4b, 5b) an je einen der zweiten Fluidkanäle (6, 7) angrenzt und wobei einer der ersten Fluidkanäle (4b) unterhalb des daran angrenzenden zweiten Fluidkanals (6) angeordnet ist und der andere der ersten Fluidkanäle (5b) oberhalb des daran angrenzenden Fluidkanals (7) angeordnet ist.

11. Substrat (1) nach einem der vorangegangenen Ansprüche,
wobei das Substrat (1) mindestens einen Anschluss (17, 18) aufweist, wobei das Substrat (1) derart ausgebildet ist, dass über den Anschluss (17, 18) Gas in das Flüssigkeitsreservoir (4, 5) eintreten, insbesondere gepumpt werden, kann,
insbesondere wobei das Substrat (1) mindestens einen ersten Anschluss (17) und einen zweiten Anschluss (18) aufweist, wobei das Substrat (1) derart ausgebildet ist, dass über den ersten Anschluss (17) Gas in eines der Flüssigkeitsreservoire (4) eintreten, insbesondere gepumpt werden, kann und über den zweiten Anschluss (18) Gas in ein anderes der Flüssigkeitsreservoire (5) eintreten, insbesondere gepumpt werden, kann.

12. Substrat (1) nach einem der Ansprüche 1 bis 11,
wobei das Substrat (1) ein Verdrängungselement (36) umfassend eine Kammer (36a), die keine fluidische Verbindung mit dem Flüssigkeitsreservoir (4, 5) aufweist, und mindestens einen Anschluss (37) umfasst,
wobei das Verdrängungselement (36) derart ausgebildet ist, dass durch Pumpen von Gas und/oder Flüssigkeit in die Kammer (36a) das Volumen der Kammer (36a) erhöht wird, und
wobei das Substrat (1) derart ausgebildet und angeordnet ist, dass über den Anschluss (37) Gas und/oder Flüssigkeit in die Kammer (36a) gepumpt werden kann und dass das Volumen des Flüssigkeitsreservoirs (4, 5) reduziert wird, wenn durch das Pumpen von Gas und/oder Flüssigkeit in die Kammer (36a) das Volumen der Kammer (36a) erhöht wird.

13. System (44) umfassend das Substrat (1) nach einem der vorangegangenen Ansprüche, wobei das System (1) ein Pumpensystem (53) umfasst, das zum Anschließen an das Substrat (1), insbesondere an einen oder mehrere der Anschlüsse (17, 18, 37) des Substrats, ausgebildet ist, insbesondere umfassend mindestens eine Pumpe (47, 52), und wobei das System optional eine Steuervorrichtung (54) zum Ansteuern des Pumpensystems (53) umfasst.

14. Verfahren zur Verwendung des Substrats (1) nach einem der Ansprüche 1 bis 12 oder des Systems (44) nach Anspruch 13, umfassend
Anlegen von Druck an das Fluidsystem (2), insbesondere mittels des Pumpensystems (53), derart, dass mindestens eines der Sperrelemente (13, 13-1, 13-2, 15) eine Durchlassstellung einnimmt und Flüssigkeit von einem der Flüssigkeitsreservoire (4, 5) in die Probenkammer (3) hinein und/oder Flüssigkeit von der Probenkammer (3) in eines der Flüssigkeitsreservoire (4, 5) transportiert wird, und optional derart, dass zugleich mindestens ein anderes der Sperrelemente (13, 13-1, 13-2, 15) eine Durchlassstellung einnimmt und/oder ein anderes der Sperrelemente (13, 13-1, 13-2, 15) eine Sperrstellung einnimmt.

15. Verfahren nach Anspruch 14, wobei das Anlegen von Druck derart erfolgt, dass eine erste Flüssigkeit, beispielsweise eine Spülflüssigkeit oder ein Puffer, aus einem ersten Flüssigkeitsreservoir (4-1) in die Probenkammer (3) transportiert und dadurch eine zweite Flüssigkeit aus der Probenkammer (3) verdrängt wird, dass die zweite Flüssigkeit in ein zweites Flüssigkeitsreservoir (5) transportiert wird und dass anschließend eine dritte Flüssigkeit aus einem dritten Flüssigkeitsreservoir (4-2) in die Probenkammer (3) transportiert und dadurch die erste Flüssigkeit aus der Probenkammer (3) verdrängt wird, insbesondere derart, dass die erste Flüssigkeit in das zweite Flüssigkeitsreservoir (5) oder in ein viertes Flüssigkeitsreservoir transportiert wird.

## Geänderte Patentansprüche

### Geänderte Patentansprüche gemäss Regel 137(2) EPÜ.

1. Substrat (1) zur Untersuchung von Proben (24), insbesondere Zellen oder Molekülen,
wobei das Substrat (1) ein Fluidsystem (2) umfasst, das eine in dem Substrat (1) ausgebildete Probenkammer (3) zur Aufbewahrung und Untersuchung von Proben (24) und mindestens ein mit der Probenkammer (3) fluidisch verbundenes Flüssigkeitsreservoir (4, 5) umfasst, und
wobei das Substrat (1) mindestens ein passives Sperrelement (13, 15) umfasst, das eine Sperrstellung und eine Durchlassstellung einnehmen kann, wobei in der Sperrstellung ein Flüssigkeitsaustausch zwischen der Probenkammer (3) und dem Flüssigkeitsreservoir (4, 5) gesperrt ist.

2. Substrat (1) nach Anspruch 1, wobei das passive Sperrelement (13, 15) derart ausgebildet ist, dass es abhängig von den Druckverhältnissen in dem Fluidsystem (2) die Sperrstellung oder die Durchlassstellung einnimmt.

3. Substrat (1) nach Anspruch 1 oder 2, wobei in dem Substrat (1) keine aktiven Komponenten angeordnet sind, insbesondere keine aktiven Ventile und/oder Pumpen.

4. Substrat (1) nach einem der vorangegangenen Ansprüche,
wobei das Flüssigkeitsreservoir (4, 5) derart verschließbar ist, dass es keine direkte Verbindung nach außen hat und/oder wobei die Probenkammer (3) derart verschließbar ist, dass sie keine direkte Verbindung nach außen hat, insbesondere, wobei das gesamte Fluidsystem (2) derart verschließbar ist, dass es keine direkte Verbindung nach außen hat.

5. Substrat (1) nach einem der vorangegangenen Ansprüche, wobei das Substrat (1) mindestens zwei passive Sperrelemente (13, 15) umfasst, insbesondere wobei das Substrat (1) derart ausgebildet ist, dass die Sperrelemente (13, 15) unabhängig voneinander die Sperrstellung oder die Durchlassstellung einnehmen.

6. Substrat (1) nach einem der vorangegangenen Ansprüche,
wobei das Fluidsystem (2) mehrere mit der Probenkammer (3) fluidisch verbundene Flüssigkeitsreservoire (4, 5) umfasst und das Substrat (1) mehrere passive Sperrelemente (13, 15) umfasst,
wobei in der Sperrstellung der Sperrelemente (13, 15) jeweils ein Flüssigkeitsaustausch zwischen der Probenkammer (3) und einem der Flüssigkeitsreservoire (4, 5) gesperrt ist und/oder ein Flüssigkeitsaustausch zwischen den Flüssigkeitsreservoiren (4, 5) gesperrt ist,
insbesondere wobei das Substrat (1) derart ausgebildet ist, dass die Sperrelemente (13, 15) unabhängig voneinander die Sperrstellung oder die Durchlassstellung einnehmen.

7. Substrat (1) nach einem der vorangegangenen Ansprüche,
wobei das Fluidsystem (2) einen ersten Fluidkanal (4b, 5b), der Teil des Flüssigkeitsreservoirs (4, 5) oder mit diesem unmittelbar fluidisch verbunden ist, und einen zweiten Fluidkanal (6, 7) umfasst, der mit der Probenkammer (3) unmittelbar fluidisch verbunden ist,
wobei der erste Fluidkanal (4b, 5b) unmittelbar an den zweiten Fluidkanal (6, 7) angrenzt und das Sperrelement (13, 15) zwischen dem ersten Fluidkanal (4b, 5b) und dem zweiten Fluidkanal (6, 7) angeordnet ist,
insbesondere, wobei das Fluidsystem (2) mindestens eine erste Probenkammer (3-1) und eine zweite Probenkammer (3-2) und mindestens zwei zweite Fluidkanäle (6-1, 6-2, 7-1, 7-2) umfasst, wobei der erste Fluidkanal (4b, 5b) über einen der zweiten Fluidkanäle (6-1, 7-1) mit der ersten Probenkammer (3-1) und über einen anderen der zweiten Fluidkanäle (6-2, 7-2) mit der zweiten Probenkammer (3-2) verbunden ist.

8. Substrat (1) nach einem der vorangegangenen Ansprüche,
wobei das Fluidsystem (2) mindestens ein erstes Sperrelement (13-1) und ein zweites Sperrelement (13-2), mindestens ein erstes Flüssigkeitsreservoir (4-1) und ein zweites Flüssigkeitsreservoir (4-2), mindestens zwei erste Fluidkanäle (4b-1, 4b-2) und einen zweiten Fluidkanal (6), der mit der Probenkammer (3) unmittelbar fluidisch verbunden ist, umfasst,
wobei einer der ersten Fluidkanäle (4b-1) Teil des ersten Flüssigkeitsreservoirs (4-1) oder mit diesem unmittelbar fluidisch verbunden ist und ein anderer der ersten Fluidkanäle (4b-2) Teil des zweiten Flüssigkeitsreservoirs (4-2) oder mit diesem unmittelbar fluidisch verbunden ist, und
wobei die ersten Fluidkanäle (4b-1, 4b-2) unmittelbar an den zweiten Fluidkanal (6) angrenzen und das erste Sperrelement (13-1) zwischen dem einen der ersten Fluidkanäle (4b-1) und dem zweiten Fluidkanal (6) angeordnet ist und das zweite Sperrelement (13-2) zwischen dem anderen der ersten Fluidkanäle (4b-2) und dem zweiten Fluidkanal (6) angeordnet ist,
insbesondere, wobei das Fluidsystem (2) mindestens eine erste Probenkammer (3-1) und eine zweite Probenkammer (3-2) und mindestens zwei zweite Fluidkanäle (6-1, 6-2) umfasst, wobei mindestens einer der ersten Fluidkanäle (4b-1, 4b-2) über einen der zweiten Fluidkanäle (6-1) mit der ersten Probenkammer (3-1) und über einen anderen der zweiten Fluidkanäle (6-2) mit der zweiten Probenkammer (3-2) verbunden ist.

9. Substrat (1) nach einem der vorangegangenen Ansprüche,
wobei das Fluidsystem (2) mindestens ein erstes Sperrelement (13) und ein zweites Sperrelement (15), mindestens ein erstes Flüssigkeitsreservoir (4) und ein zweites Flüssigkeitsreservoir (5), mindestens zwei erste Fluidkanäle (4b, 5b) und mindestens zwei zweite Fluidkanäle (6, 7), die mit der Probenkammer (3) unmittelbar fluidisch verbunden sind, umfasst,
wobei einer der ersten Fluidkanäle (4b) Teil des ersten Flüssigkeitsreservoirs (4) oder mit diesem unmittelbar fluidisch verbunden ist und unmittelbar an einen der zweiten Fluidkanäle (6) angrenzt und dazwischen das erste Sperrelement (13) angeordnet ist,
wobei ein anderer der ersten Fluidkanäle (5b) Teil des zweiten Flüssigkeitsreservoirs (5) oder mit diesem unmittelbar fluidisch verbunden ist und unmittelbar an einen anderen der zweiten Fluidkanäle (7) angrenzt und dazwischen das zweite Sperrelement (15) angeordnet ist,
insbesondere wobei das erste Sperrelement (13) nur eine Durchlassrichtung von dem ersten Flüssigkeitsreservoir (4) in die Probenkammer (3) und das zweite Sperrelement (15) nur eine Durchlausrichtung aus der Probenkammer (3) in Richtung des zweiten Flüssigkeitsreservoirs (5) hat, und/oder
insbesondere wobei das erste Sperrelement (13) oberhalb des an das erste Sperrelement (13) angrenzenden ersten Fluidkanals (4b) und unterhalb des an das erste Sperrelement (13) angrenzenden zweiten Fluidkanals (6) angeordnet ist und/oder wobei das zweite Sperrelement (15) unterhalb des an das zweite Sperrelement (15) angrenzenden ersten Fluidkanals (5b) und oberhalb des an das zweite Sperrelement (15) angrenzenden zweiten Fluidkanals (7) angeordnet ist.

10. Substrat (1) nach einem der Ansprüche 7 bis 9,
wobei der erste Fluidkanal (4b, 5b) und der zweite Fluidkanal (6, 7), in einem Bereich, in dem der erste Fluidkanal (4b, 5b) unmittelbar an den zweiten Fluidkanal (6, 7) angrenzt, in Draufsicht auf das Substrat (1) einander überlappend angeordnet sind und der erste Fluidkanal (4b, 5b) oberhalb oder unterhalb des zweiten Fluidkanals (6, 7) angeordnet ist,
insbesondere, wobei das Substrat (1) mindestens zwei erste Fluidkanäle (4b, 5b) und zwei zweite Fluidkanäle (6, 7) aufweist, wobei je einer der ersten Fluidkanäle (4b, 5b) an je einen der zweiten Fluidkanäle (6, 7) angrenzt und wobei einer der ersten Fluidkanäle (4b) unterhalb des daran angrenzenden zweiten Fluidkanals (6) angeordnet ist und der andere der ersten Fluidkanäle (5b) oberhalb des daran angrenzenden Fluidkanals (7) angeordnet ist.

11. Substrat (1) nach einem der vorangegangenen Ansprüche,
wobei das Substrat (1) mindestens einen Anschluss (17, 18) aufweist, wobei das Substrat (1) derart ausgebildet ist, dass über den Anschluss (17, 18) Gas in das Flüssigkeitsreservoir (4, 5) eintreten kann,
insbesondere wobei das Substrat (1) mindestens einen ersten Anschluss (17) und einen zweiten Anschluss (18) aufweist, wobei das Substrat (1) derart ausgebildet ist, dass über den ersten Anschluss (17) Gas in eines der Flüssigkeitsreservoire (4) eintreten kann und über den zweiten Anschluss (18) Gas in ein anderes der Flüssigkeitsreservoire (5) eintreten kann.

12. Substrat (1) nach einem der Ansprüche 1 bis 11,
wobei das Substrat (1) ein Verdrängungselement (36) umfassend eine Kammer (36a), die keine fluidische Verbindung mit dem Flüssigkeitsreservoir (4, 5) aufweist, und mindestens einen Anschluss (37) umfasst,
wobei die Kammer des Verdrängungselements in dem Flüssigkeitsreservoir oder an das Flüssigkeitsreservoir angrenzend in einer Aufnahme im Substrat angeordnet ist,
wobei das Verdrängungselement (36) derart ausgebildet ist, dass durch Pumpen von Gas und/oder Flüssigkeit in die Kammer (36a) das Volumen der Kammer (36a) erhöht wird, und
wobei das Substrat (1) derart ausgebildet und angeordnet ist, dass über den Anschluss (37) Gas und/oder Flüssigkeit in die Kammer (36a) gepumpt werden kann und dass das Volumen des Flüssigkeitsreservoirs (4, 5) reduziert wird, wenn durch das Pumpen von Gas und/oder Flüssigkeit in die Kammer (36a) das Volumen der Kammer (36a) erhöht wird.

13. System (44) umfassend das Substrat (1) nach einem der vorangegangenen Ansprüche, wobei das System (1) ein Pumpensystem (53) umfasst, das zum Anschließen an das Substrat (1) ausgebildet ist und wobei das System optional eine Steuervorrichtung (54) zum Ansteuern des Pumpensystems (53) umfasst.

14. Verfahren zur Verwendung des Substrats (1) nach einem der Ansprüche 1 bis 12 oder des Systems (44) nach Anspruch 13, umfassend
Anlegen von Druck an das Fluidsystem (2), insbesondere mittels des Pumpensystems (53), derart, dass mindestens eines der Sperrelemente (13, 13-1, 13-2, 15) eine Durchlassstellung einnimmt und Flüssigkeit von einem der Flüssigkeitsreservoire (4, 5) in die Probenkammer (3) hinein und/oder Flüssigkeit von der Probenkammer (3) in eines der Flüssigkeitsreservoire (4, 5) transportiert wird, und optional derart, dass zugleich mindestens ein anderes der Sperrelemente (13, 13-1, 13-2, 15) eine Durchlassstellung einnimmt und/oder ein anderes der Sperrelemente (13, 13-1, 13-2, 15) eine Sperrstellung einnimmt.

15. Verfahren nach Anspruch 14, wobei das Anlegen von Druck derart erfolgt, dass eine erste Flüssigkeit aus einem ersten Flüssigkeitsreservoir (4-1) in die Probenkammer (3) transportiert und dadurch eine zweite Flüssigkeit aus der Probenkammer (3) verdrängt wird, dass die zweite Flüssigkeit in ein zweites Flüssigkeitsreservoir (5) transportiert wird und dass anschließend eine dritte Flüssigkeit aus einem dritten Flüssigkeitsreservoir (4-2) in die Probenkammer (3) transportiert und dadurch die erste Flüssigkeit aus der Probenkammer (3) verdrängt wird, insbesondere derart, dass die erste Flüssigkeit in das zweite Flüssigkeitsreservoir (5) oder in ein viertes Flüssigkeitsreservoir transportiert wird.
